(19) Europäisches Patentamt
European Patent Office
Office européen des brevets

(11) **EP 4 231 316 A1**

(12) **EUROPÄISCHE PATENTANMELDUNG**

(43) Veröffentlichungstag:
**23.08.2023 Patentblatt 2023/34**

(21) Anmeldenummer: **23156813.0**

(22) Anmeldetag: **15.02.2023**

(51) Internationale Patentklassifikation (IPC):
**G21K 1/02** (2006.01)   **B23K 26/0622** (2014.01)
**B23K 26/53** (2014.01)   **C03C 23/00** (2006.01)

(52) Gemeinsame Patentklassifikation (CPC):
**G21K 1/025; B23K 26/0006; B23K 26/0624;**
**B23K 26/0853; B23K 26/53; C03C 23/0025;**
B23K 2103/54

(84) Benannte Vertragsstaaten:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Benannte Erstreckungsstaaten:
**BA**
Benannte Validierungsstaaten:
**KH MA MD TN**

(30) Priorität: **22.02.2022 DE 102022104180**

(71) Anmelder:
• **SCHOTT AG**
**55122 Mainz (DE)**
• **Schott North America, Inc.**
**Rye Brook, NY 10573 (US)**

(72) Erfinder:
• **SOHR, Oliver**
**55116 Mainz (DE)**
• **LEUGNER, Sebastian**
**64579 Gernsheim (DE)**
• **TREIS, Philipp**
**55411 Bingen am Rhein (DE)**
• **TABOR, Kevin**
**Webster, 01570 (US)**
• **DARGIE, Michael**
**Southbridge, 01550 (US)**

(74) Vertreter: **Schott Corporate IP**
**Hattenbergstraße 10**
**55122 Mainz (DE)**

(54) **ABSCHIRMMASKE FÜR IONISIERENDE STREUSTRAHLUNG UND VERFAHREN ZU DESSEN HERSTELLUNG**

(57) Der Erfindung liegt die Aufgabe zugrunde, ein verbessertes Gitter zur Abschirmung von Röntgen-Streustrahlung für eine bildgebende Röntgenvorrichtung zur Verfügung zu stellen. Dazu ist ein Abschirmgitter (1) gegen Röntgen-Streustrahlung vorgesehen, umfassend
- eine Scheibe (3) mit einer ersten Seite (5) und einer der ersten Seite (5) gegenüberliegenden zweiten Seite (7), wobei die Scheibe (3)
- eine Anordnung von Vertiefungen (15) aufweist, welche zur zweiten Seite (7) der Scheibe (3) hin geöffnet sind, und wobei
- die Scheibe (3) ein Gitter (9) aus zur ersten Seite (5) hin geöffneten Gräben (11) aufweist, wobei
- die Gräben (11) mit einem Röntgenstrahlungs-absorbierenden Material (13) gefüllt sind, und wobei
- die Gräben (11) von einer der Seiten (5, 7) aus betrachtet zwischen den Vertiefungen (15) und in einem Abstand zu den Vertiefungen (15) verlaufen, so dass zwischen den Vertiefungen und den Gräben (11) Wandungen (19) verbleiben.

Fig. 2

**Beschreibung**

Die Erfindung betrifft allgemein bildgebende Verfahren mit ionisierender Strahlung, wie insbesondere bildgebende Röntgenverfahren. Insbesondere betrifft die Erfindung Masken zur Abschirmung eines Strahlungsdetektors vor gestreuter ionisierender Strahlung.

Ein bekanntes bildgebendes Verfahren mit ionisierender Strahlung ist die Computertomographie, wie sie als radiologisches Verfahren in der Diagnostik eingesetzt wird. Hierbei wird das zu untersuchende Objekt in verschiedenen Ebenen und Richtungen mit einem Röntgen-Fächerstrahl durchleuchtet. Aus den ortsaufgelöst aufgezeichneten Signalen kann dann mittels eines Rechners ein dreidimensionales Modell des Objekts rekonstruiert werden.

Eine Form der Computertomographie ist die digitale Volumentomographie (DVT). Bei diesem Verfahren wird das Objekt mit der Strahlung einer möglichst punktförmigen Röntgenquelle durchleuchtet und mit einem Matrixdetektor aufgezeichnet.

Für das Signal/Rausch-Verhältnis und damit die Auflösung und den Kontrast der Tomogramme ist es von Vorteil, wenn Streustrahlung vor dem Detektor abgeblockt wird. Hierzu ist es bekannt, Gitter zu verwenden, die unter einem Winkel zum direkten geradlinigen Pfad zur Röntgenquelle am Detektor eintreffende Strahlung absorbieren soll. Eine derzeit verwendete Ausführungsform eines solchen Gitters sieht einen Stapel von Bleistreifen vor, zwischen denen sich als Abstandhalter Papierstreifen befinden. Der Nachteil ist hier unter anderem, dass eine solche Struktur nur in einer Ebene, nämlich senkrecht zu den Oberflächen der Bleistreifen Streustrahlung unterdrückt. Zudem ist eine solche Struktur mechanisch nicht sehr stabil und kann sich leicht dauerhaft deformieren. Auch ermöglicht der Aufbau im Allgemeinen keine sehr feine Strukturierung, was wiederum auch die Ortsauflösung des Tomographen beeinflussen kann.

Aus der WO 2007/034352 A2 ist ein Röntgenabsorbtionsgitter bekannt, bei dem in ähnlicher Weise Lamellen aus röntgenabsorbierendem Material, wie Wolfram oder Molybdän in einem Hartschaummaterial eingelassen sind und durch das Hartschaummaterial mechanisch stabilisiert werden.

Die US2016163408 A1 beschreibt die Herstellung eines Röntgenabsorptionsgitters mittels eines Silizium-Substrats. Dazu werden in das Silizium-Substrat durch Ätzen Aushöhlungen hergestellt, die dann galvanisch mit röntgenabsorbierendem Metall aufgefüllt werden.

Das aus der US 5581592 A bekannte Röntgenabsorbtionsgitter wird hergestellt, indem in ein Substrat, vorzugsweise aus Kunststoff, Kanäle durch Sägen eingefügt werden. Dazu können Sägeblätter verwendet werden, wie sie in zum Zerteilen von Siliziumwafern bei der Chipfertigung eingesetzt werden. In die Kanäle wird dann eine röntgenabsorbierende Legierung durch Aufschmelzen der Legierung eingefügt. Dazu muss das Substrat der Schmelztemperatur ohne Erweichen widerstehen können.

Der Erfindung liegt die Aufgabe zugrunde, ein verbessertes Gitter zur Abschirmung von ionisierender Streustrahlung, insbesondere Röntgenstrahlung, zur Verfügung zu stellen. Diese Aufgabe wird durch den Gegenstand der unabhängigen Ansprüche gelöst. Vorteilhafte Ausgestaltungen der Erfindung sind in den jeweiligen abhängigen Ansprüchen angegeben.

Demgemäß sieht die Erfindung ein Abschirmgitter gegen Röntgen-Streustrahlung, insbesondere für eine bildgebende Röntgenvorrichtung, beispielsweise einen Röntgen-Computertomographen vor, umfassend

- eine Scheibe mit einer ersten Seite und einer der ersten Seite gegenüberliegenden zweiten Seite, wobei die Scheibe
- eine Anordnung von Vertiefungen aufweist, welche zur zweiten Seite der Scheibe hin geöffnet sind, und wobei
- die Scheibe ein Gitter aus zur ersten Seite hin geöffneten Gräben aufweist, wobei
- die Gräben mit einem Röntgenstrahlungs-absorbierenden Material gefüllt sind, und wobei
- die Gräben von einer der Seiten aus betrachtet zwischen den Vertiefungen und in einem Abstand zu den Vertiefungen verlaufen, so dass zwischen den Vertiefungen und den Gräben Wandungen verbleiben.

In Richtung auf eine der Seiten der Scheibe betrachtet sind die Vertiefungen somit zwischen den Gräben angeordnet. Die direkte Röntgenstrahlung kann an den Positionen der Vertiefungen leicht durch die Scheibe hindurchtreten. Da um die Vertiefungen herum die Wandungen aus dem Material der Scheibe angeordnet sind, wird der Scheibe hinreichend mechanische Stabilität verliehen, auch wenn das Röntgenstrahlungs-absorbierende Material in den Gräben selbst nicht mechanisch belastbar ist. Da sich die Vertiefungen und Gräben zu unterschiedlichen Seiten der Scheibe hin öffnen und die Vertiefungen somit zur Seite, zu der sich die Gräben öffnen, geschlossen sind, kann in einfacher Weise vermieden werden, dass beim Befüllen der Gräben mit dem Röntgenstrahlungs-absorbierenden Material auch gleichzeitig die Vertiefungen befüllt werden. Als Röntgenstrahlungs-absorbierendes Material wird im Sinne dieser Offenbarung ein Material bezeichnet, dessen Röntgenabsorptionskoeffizient mindestens um einen Faktor 3 größer ist, als der Röntgenabsorbtionskoeffizient des Materials der Scheibe für Röntgenstrahlung mit einer Energie von 69,5 keV. Gemäß einer alternativen oder zusätzlichen, bevorzugten Ausführungsform ist das Röntgenstrahlungs-absorbierende Material ein Material mit einer Dichte, die mindestens viermal größer ist als die Dichte des Materials der Scheibe. Für die Wirkung als Röntgenstrahlungs-absorbierendes Material ist besonders auch der Gehalt an Elementen mit hoher Kernladungszahl wichtig. Gemäß noch einer alternativen oder zusätzlichen Ausführungsform ist ein Röntgenstrahlungs-absorbierendes Material ein Material, welches zu mindestens 10 Gewichtsprozent Elemente mit einer Kernladungszahl von mindestens Z=56, vorzugsweise zu mindestens 25 Gewichtsprozent, besonders bevorzugt zu einem Anteil von mindestens 50 Gewichtsprozent solche Elemente mit Z≥56 aufweist. Im Sinne dieser Offenbarung fallen unter Röntgenstrahlungs-absorbierenden Materialien auch allgemein Materialien, welche ionisierende Strahlung, insbesondere dabei elektromagnetische Strahlung, absorbieren. Solche Materialien haben auch typischerweise eine hohe Absorbtionswirkung gegen partikuläre ionisierende Strahlung. Daher betrifft diese Offenbarung allgemein das genannte Abschirmgitter gegen ionisierende Streustrahlung. In diesem Sinne ist der Begriff eines Röntgenstrahlungs-absorbierenden Materials eine Vereinfachung für alle Materialien, die geeignet sind, hochenergetische Strahlen zu absorbieren. Unter Röntgenstrahlungs-absorbierende Materialien fallen weiterhin auch röntgenopake Materialien.

Die Erfindung wird nachfolgend anhand der Figuren näher erläutert. Dabei verweisen gleiche Bezugszeichen in den Figuren auf jeweils gleiche oder entsprechende Elemente.

Kurzbeschreibung der Figuren:

| Fig. 1 | zeigt schematisch einen Computertomograph mit einem Abschirmgitter. |
| Fig. 2 | zeigt ein Abschirmgitter im Querschnitt. |
| Fig. 3 | zeigt ein Abschirmgitter in Aufsicht. |
| Fig. 4 | zeigt eine Variante des Abschirmgitters. |
| Fig. 5 | zeigt im Querschnitt eine Variante des Abschirmgitters mit auf eine Punktquelle ausgerichteten Vertiefungen. |
| Fig. 6 | zeigt eine Laser-Bearbeitungsvorrichtung zum Einfügen von filamentförmigen Schädigungen in die Scheibe. |
| Fig. 7 | zeigt im Querschnitt eine Scheibe mit eingefügten filamentförmigen Schädigungen. |
| Fig. 8 | zeigt die Scheibe nach dem Ätzen. |
| Fig. 9 | zeigt die Scheibe nach dem Auffüllen der Gräben mit einem Röntgenstrahlungsabsorbierenden Material. |
| Fig. 10 | zeigt eine Variante der Ausführungsform nach Fig. 9 mit einer ausgedünnten Scheibe. |
| Fig. 11 | zeigt schematisch ein Querschnitt durch ein Röntgenstrahlungs-absorbierendes Material. |
| Fig. 12 | zeigt eine Partikelgrößenverteilung für ein gemahlenes Glaslot. |
| Fig. 13 | zeigt Partikelgrößenverteilungen für gemahlenes Glas, zwei Metallstäube und deren Mischung. |
| Fig. 14 | stellt eine Weiterbildung der in Fig. 2 gezeigten Ausführungsform dar. |
| Fig. 15 | ist eine weitere Weiterbildung der in Fig. 2 gezeigten Ausführungsform. |
| Fig. 16 bis Fig. 19 | zeigen Verfahrensschritte zur Befüllung der Gräben des Abschirmgitters mit röntgenabsorbierendem Material. |
| Fig. 20 bis Fig. 23 | zeigen Verfahrensschritte zur Befüllung gemäß einer weiteren Ausführungsform des Herstellungsverfahrens. |
| Fig. 24 und Fig. 25 | zeigen Verfahrensschritte, um Zwischenräume in der Befüllung der Gräben aufzufüllen. |
| Fig. 26 und Fig. 27 | zeigen eine Variante des Verfahrens, bei welcher die Befüllung der Gräben mit Metallpulvern unterschiedlicher Körnung und Partikelform erfolgt. |
| Fig. 28 bis Fig. 30 | zeigen eine Ausführungsform der Befüllung der Gräben, bei welcher die Gräben von einer Dispersion durchströmt werden. |
| Fig. 31 | zeigt eine Anordnung zur Laserbearbeitung für die Herstellung der Gräben. |

Ausführliche Beschreibung der Figuren

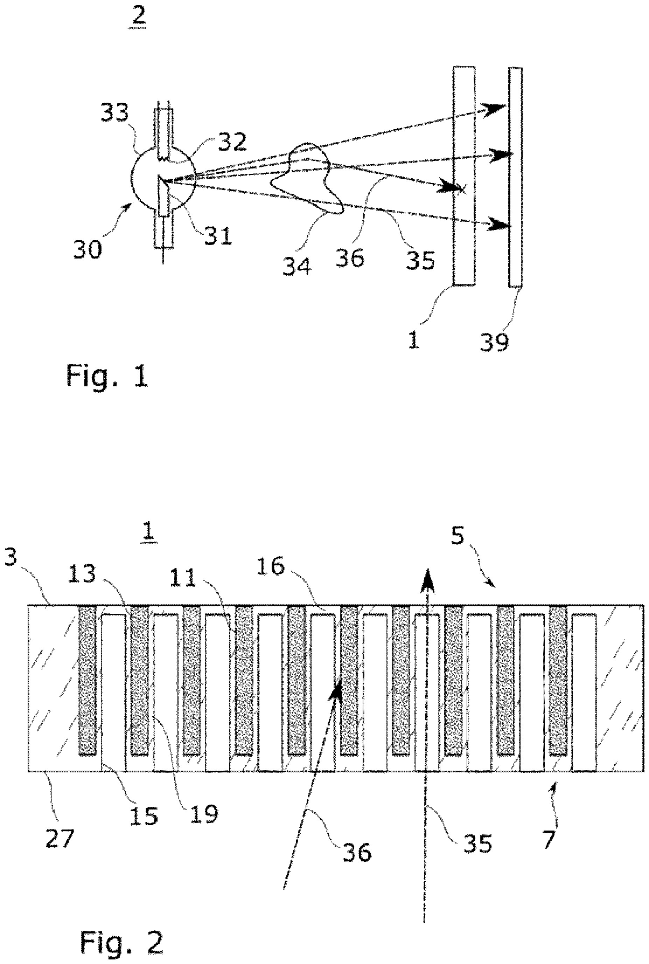

Die Erfindung betrifft neben dem Abschirmgitter auch eine bildgebende Röntgenvorrichtung, wie etwa einen Computertomographen. Die bildgebende Röntgenvorrichtung umfasst allgemein eine Röntgenquelle, einen Röntgendetektor und einem vor dem Röntgendetektor zur Detektion der von der Röntgenquelle emittierten Röntgenstrahlung angeordnetes Abschirmgitter 1. Die Funktion eines Abschirmgitters 1, wie es die Erfindung vorsieht, wird anhand von Fig. 1 erläutert. Fig. 1 zeigt einen schematischen Aufbau eines bildgebenden Röntgengeräts 2. Dieses umfasst in der dargestellten Ausführungsform eine Röntgenquelle, insbesondere eine Röntgenröhre 30 mit einem Vakuumkolben 33 und darin angeordnet eine Anode 31 und eine Kathode 32. Im Betrieb werden von der Anode Röntgenstrahlen emittiert. Ein zu untersuchendes Objekt, beispielsweise ein Patient oder ein Körperteil werden zwischen der Röntgenröhre 30 und einem Röntgendetektor 39 angeordnet.

Die Erfindung ist besonders geeignet für die sogenannte Kegelstrahl-Computertomographie. Bei diesem Verfahren wird kein sequenzieller Scan mit einem rotierenden Fächerstrahl durchgeführt, sondern vielmehr ein von der Röntgenröhre 30 ausgehender Strahlkegel mit einem Matrix-Detektor erfasst. Für das Tomogramm geeignete Daten können aus geradlinig durch das zu untersuchende Objekt hindurchgehende Röntgenstrahlen 35 gewonnen werden. Gestreute Röntgenstrahlen hingegen enthalten keine Ortsinformation und erhöhen lediglich das Rauschen. Wie anhand der von Fig. 1 zu erkennen ist, treffen gestreute Röntgenstrahlen unter einem Winkel gegenüber dem geradlinigen Pfad zwischen Röntgenröhre und Detektor auf dem Röntgendetektor 39 auf. Das Abschirmgitter 1 soll nun die geradlinig von der Röntgenröhre 30 kommenden Röntgenstrahlen 35 möglichst ohne Abschwächung hindurchlassen, gestreute Röntgenstrahlen 34 hingegen absorbieren. Dies wird durch Durchgänge erreicht, die durch die Zwischenräume des Gitters aus röntgenabsorbierendem Material definiert werden und gegenüber einem geradlinigen Durchgang nur Strahlen unter einem geringen Winkel hindurchlassen.

Ein Abschirmgitter 1 ist im Querschnitt in Fig. 2 dargestellt. Das Abschirmgitter 1 umfasst eine Scheibe 3 als Träger oder Grundelement. Gemäß einer besonders bevorzugten Ausführungsform der Erfindung und ohne Beschränkung auf das dargestellte Beispiel ist die Scheibe 3 eine Glasscheibe. Glas wird als Material unter anderem deshalb besonders bevorzugt, da dieses nicht duktil ist und sich aufgrund einer mechanischen Einwirkung somit nicht dauerhaft verformen lässt. Damit werden Änderungen in den kollimierenden Eigenschaften, beziehungsweise der Selektivität für direkte gegenüber gestreuter Röntgenstrahlung vermieden. Ein weiterer Grund ist, dass Glas mittlerweile sehr fein strukturierbar ist, was ebenfalls eine Herstellung eines Abschirmgitters mit hoher Selektivität erleichtert. Ein weiterer Vorteil ist, dass durch Auswahl einer geeigneten Glasart die Temperaturausdehnung des Glases abgestimmt werden kann. Damit können beispielsweise temperaturbedingte Verschiebungen des Gitters gegenüber den Pixeln eines Röntgendetektors 39 minimiert werden. Dies gilt auch für vergleichsweise große Dimensionen. Daher ist gemäß einer Weiterbildung, ohne Beschränkung auf bestimmte Materialien des Abschirmgitters vorgesehen, dass das Abschirmgitter 1 eine Fläche von mindestens 0,25 m$^2$ aufweist. Insbesondere kann die Fläche sogar mindestens 1/3 m$^2$ betragen. So ist gemäß einem Ausführungsbeispiel ein Abschirmgitter mit einer Abmessung von 600 mm $\times$ 600 mm vorgesehen.

Die Scheibe 3 weist zwei gegenüberliegende Seiten 5, 7 auf. Vorzugsweise ist die Scheibe 3 planparallel ausgebildet, so dass die gegenüberliegenden Seiten 5, 7 ebenfalls parallel verlaufen. Wie anhand von Fig. 2 ersichtlich sind in beide Seiten 5, 7 Vertiefungen eingefügt. Die in der ersten Seite 5 eingefügten Vertiefungen sind dabei als Gräben 11 ausgebildet. In die gegenüberliegende zweite Seite 7 sind Vertiefungen 15 eingefügt, die zwischen den Gräben 11 liegen. Die Vertiefungen 15 sind zur zweiten Seite 7 hin offen. Entsprechend sind die Gräben 11 zur gegenüberliegenden ersten Seite 5 geöffnet. Durch die Öffnung der Gräben 11 zur ersten Seite 5 können die Gräben 11 mit einem Röntgenstrahlungs-absorbierenden Material 13 befüllt werden. Da sich die Gräben 11 im Wesentlichen senkrecht in die Scheibe 3 hinein erstrecken, kann das darin befindliche Röntgenstrahlungs- absorbierende Material wirksam schräg auf die Scheibe 3 eintretende Röntgenstrahlen absorbieren. Zur Veranschaulichung ist dazu ein im Wesentlichen senkrecht eintreffender Röntgenstrahl 35 eingezeichnet, welcher die Scheibe 3 durch eine Vertiefung 15 hindurch passieren kann. Ein schräg eintreffender Röntgenstrahl 36, wie er durch Streuung an einem zu untersuchenden Objekt entsteht, wird hingegen im Röntgenstrahlungs-absorbierenden Material 13 absorbiert. Die Vertiefungen 15 dienen also dazu, auch bei einer dickeren Scheibe 3 die Wechselwirkung der Röntgenstrahlung mit dem Material der Scheibe 3, vorzugsweise Glas, zu minimieren. Die Röntgenstrahlung muss lediglich noch die im Vergleich zur Dicke der Scheibe 3 wesentlich dünnere Bodenwandung 16 passieren.

Generell ist eine Scheibe 3 bevorzugt, die eine Dicke von mindestens 2 Millimetern, vorzugsweise mindestens 3 Millimetern aufweist. Dies gestattet das Einfügen entsprechend tiefer Gräben 11 und damit eine gute Abschirmung schräg auftreffender Streustrahlung. Vorzugsweise ist die Dicke aber auch geringer als 10 mm, damit die Gräben 11 noch leicht mit röntgenabsorbierendem Material auffüllbar sind.

Zwischen den Vertiefungen 15 und den Gräben 11 sind Wandungen 19 vorhanden. Im Bereich dieser Wandungen ist zwar eine unverminderte Dicke der Scheibe 3 vorhanden, allerdings können die Wandungen 19 schmal gehalten werden. Zudem verleihen diese Wandungen der Anordnung ihre mechanische Stabilität.

Um die mechanische Stabilität weiter zu erhöhen, kann gemäß einer Weiterbildung der Erfindung auch ein Randbereich 27 der Scheibe 3 vorgesehen werden, der keine Vertiefungen 15 oder Gräben 11 aufweist. Dieser Randbereich wirkt somit wie eine stabilisierende Einfassung. Ohne Beschränkung auf das dargestellte Beispiel kann der Randbereich eine Breite aufweisen, die mindestens doppelt so breit ist, wie die Periodenlänge der Abfolge von Gräben und Vertiefungen. Die Periode der Gräben 11, beziehungsweise der Abstand der Gräben 11 gemessen von Mitte zu Mitte der Gräben 11 beträgt gemäß einer bevorzugten Ausführungsform höchstens 500 $\mu$m. Dies ist unter anderem günstig, um eine hohe Ortsauflösung bei der Bildgebung zu erreichen.

Die Anordnung von Gräben 11 und Vertiefungen 15 kann insbesondere auch auf die Pixelabstände eines Detektors abgestimmt sein. Gerade bei einem solchen Pixelmatching können auch kleine Veränderungen des Abschirmgitters 1, etwa durch mechanische Deformationen erhebliche Transmissionseinbußen zur Folge haben. Auch daher ist Glas ein besonders bevorzugter Werkstoff für die Scheibe 3. Generell sind dabei Borosilikatgläser besonders geeignet, sowohl, was die Stabilität, als auch die was die Strukturierbarkeit betrifft. Denkbar sind allerdings auch andere Materialien, wie Glaskeramiken, Keramiken oder bestimmte Kunststoffe. Neben Borosilikatglas eignet sich allgemein auch Kalk-Natron-Glas, sowie Aluminosilikatglas gut als Material für die Scheibe. Ein Kriterium für die Wahl des Materials der Scheibe 3 kann allgemein dessen linearer thermischer Ausdehnungskoeffizient sein. Dieser kann an den Ausdehnungskoeffizient des Röntgenstrahlungs- absorbierenden Materials angenähert werden, um temperaturbedingte mechanische Spannungen gering zu halten. So kann ein Kalk-Natron-Glas beispielsweise gut geeignet sein, wenn das Röntgenstrahlungs-absorbierende Material 13 einen hohen Wärmeausdehnungskoeffizient aufweist.

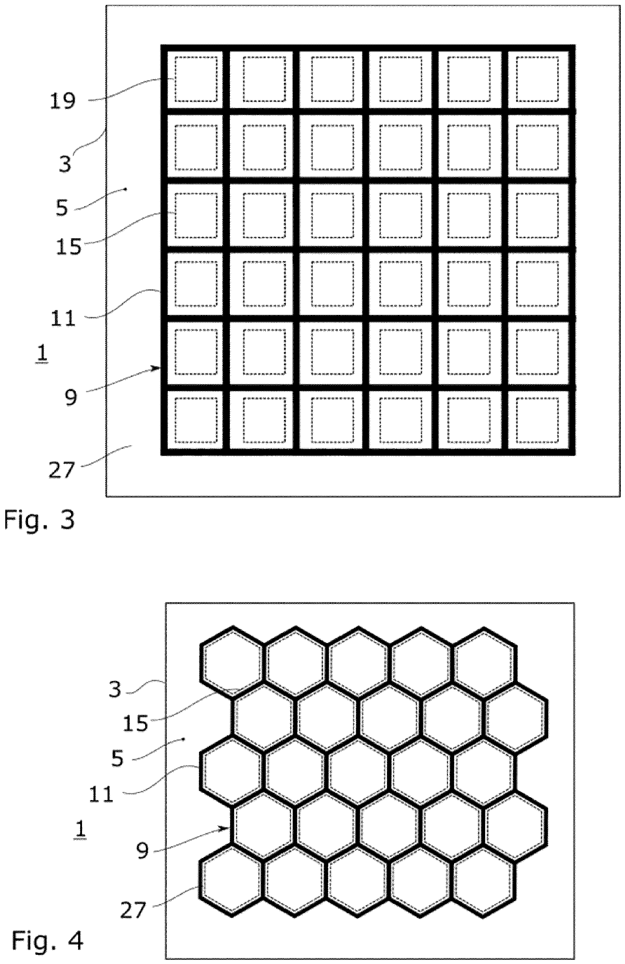

Fig. 3 zeigt das Abschirmgitter 1 in Aufsicht auf die erste Seite 5. Gemäß einer bevorzugten Weiterbildung der Erfindung ist das Gitter 9 aus Gräben 11 wie auch im dargestellten Beispiel als Kreuzgitter ausgebildet. Die sich kreuzenden Gräben 11 bilden Zellen, innerhalb derer die Vertiefungen 15 angeordnet sind. Es sind aber auch andere Anordnungen möglich. So lässt sich eine hohe Transmission für die direkte Röntgenstrahlung beispielsweise mit einem hexagonalen Gitter erreichen. Eine solche Variante mit einem hexagonalen Gitter zeigt Fig. 4.

Generell kann die Form des Gitters 9 an die Form von Pixeln des Detektors 39 angepasst werden. Neben den anhand der Fig. 3 und Fig. 4 gezeigten Beispielen sind demgemäß auch andere Formen denkbar. So kann das Gitter 9 auch rechteckige, runde, dreieckige oder beispielsweise auch oktagonale Kanäle definieren.

Die Darstellungen der Fig. 3 und Fig. 4 sind lediglich schematisch zu verstehen. In der Darstellung sind die Gräben 11 deutlich schmaler gezeichnet, als die Vertiefungen 15. In der Praxis ist es aber in der Regel günstig, ähnliche Breiten von Gräben 11 und Vertiefungen 15, beziehungsweise damit einhergehend einen im Verhältnis zur Breite der Gräben 11 geringeren Abstand von Graben zu Graben vorzusehen, um eine gute Abschirmung der Streustrahlung zu erreichen. Die Darstellung der Fig. 2 ist diesbezüglich näher an den bevorzugten Ausführungsformen. Gemäß einer Weiterbildung der Erfindung ist diesbezüglich vorgesehen, dass sich die Breite der Gräben 11 von der Breite der Vertiefungen 15 um maximal einen Faktor 2 unterscheidet.

Besonders günstig für die Transmission der direkten Röntgenstrahlung und der Abschirmung der Streustrahlung ist auch ein großes Verhältnis von Tiefe zu Breite der Gräben. Es ist insbesondere mit einem später erläuterten Verfahren möglich, auch Gräben 11 mit einem Röntgenstrahlungs-absorbierenden Material zu füllen, wenn das Verhältnis von Tiefe zu Breite 40:1 oder mehr beträgt. Bei einem zu hohen Aspektverhältnis ist eine hinreichend homogene oder vollständige Füllung aber gegebenenfalls nicht mehr gewährleistet. Daher wird es bevorzugt, das Verhältnis von Breite zu Tiefe auf 150:1 oder weniger zu begrenzen. Es ist auch hier ersichtlich, dass die Zeichnungen lediglich schematisch sind. Das Aspektverhältnis der Gräben 11 in Fig. 2 ist deutlich kleiner als 40:1. Gemäß einer alternativen oder vorzugsweise zusätzlichen Weiterbildung beträgt die Breite der Gräben 11 höchstens 100 μm, vorzugsweise höchstens 50 μm. Dies ist generell günstig für eine gleichmäßige Ausleuchtung des Detektors, indem durch die geringe Breite Abschattungen vermieden werden. In Verbindung mit einer großen Tiefe, beziehungsweise einem entsprechenden Aspektverhältnis folgt auch bei geringer Breite der Gräben 11 eine hohe Röntgenabsorbtion.

Ein weiterer alternativer oder zusätzlicher Faktor für eine gute Abschirmung von Streustrahlung und Transmission von direkter Strahlung ist die Tiefe der Gräben 11. Vorzugsweise beträgt die Tiefe der Gräben 11 mindestens 1,5 Millimeter, vorzugsweise mindestens 2 Millimeter.

Ebenfalls günstig für die Eigenschaft einer hohen Abschirmung von Streustrahlung ist es, wenn die durch die Gräben 11 des Gitters 9 definierten Durchgänge oder Kanäle möglichst lang sind. Je länger diese Kanäle im Verhältnis zu deren Breite sind, desto selektiver ist das Gitter für den Durchlass direkter Strahlung und der Abschirmung schräg auftreffender Streustrahlung. So ist gemäß noch einer Weiterbildung der Erfindung vorgesehen, dass der Mitte-zu-Mitte-Abstand zweier benachbarter Gräben kleiner als deren Tiefe ist. Vorzugsweise ist der Mitte-zu-Mitte-Abstand sogar um mindestens einen Faktor drei kleiner als die Tiefe der Gräben 11.

Schließlich sollten für eine gute Abschirmung die Wände der Gräben 11 möglichst rechtwinklig zu den Ebenen der Seiten 5, 7 verlaufen. Diesbezüglich wird es bevorzugt, wenn der Winkel der Wand 25 eines Grabens 11 zur ersten Seite 5 um weniger als 5° von einem rechten Winkel abweicht. Auch die geringen Taper-Winkel von weniger als 5° lassen sich mit dem später beschriebenen Herstellungsverfahren insbesondere in einer Glasscheibe realisieren. Dies gilt für den Spezialfall von senkrecht in die Scheibe einschneidenden Gräben 11 und Vertiefungen 15.

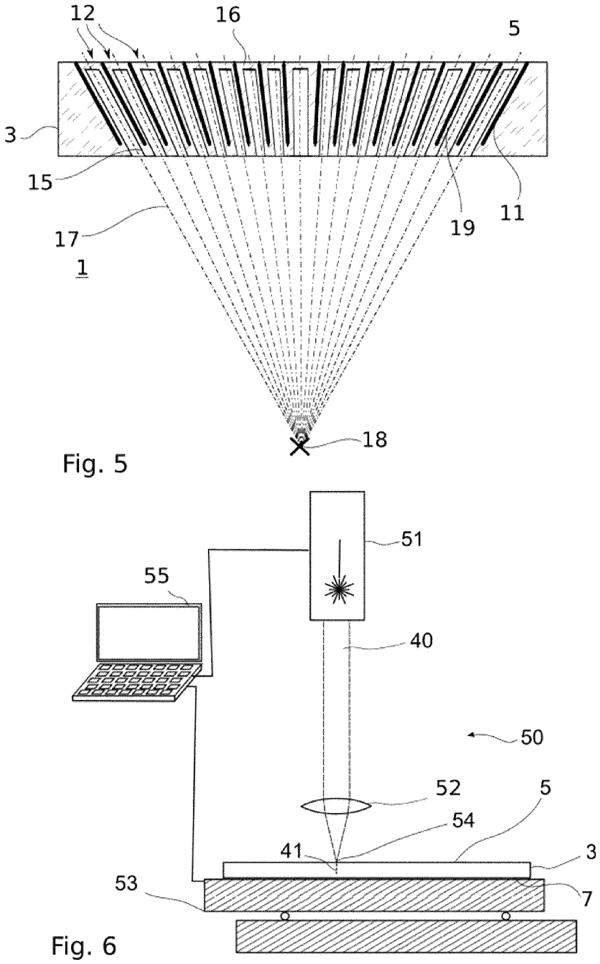

Die Gräben 11 verlaufen bei dem in Fig. 2 gezeigten Beispiel parallel zueinander in Richtung senkrecht zu den Seiten 5, 7. Eine solche Anordnung ist insbesondere dann günstig, wenn die zu detektierenden Röntgenstrahlen als Parallelstrahl-Bündel auftreffen, also etwa, wenn die Röntgenquelle weit entfernt ist. Typischerweise wird aber in einer bildgebenden Röntgenvorrichtung, wie etwa einem Computertomograph eine näher angeordnete Röntgenquelle verwendet, so dass auf das Abschirmgitter ein konisches Strahlenbündel fällt. Allgemein ist daher in einer Ausführungsform der Erfindung vorgesehen, dass die Gräben 11 variierende Neigungen aufweisen, so dass die Wandungen der Gräben 11 entlang von Richtungen verlaufen, die auf eine gemeinsame virtuelle Punktquelle weisen. Anders ausgedrückt sind die Gräben 11 derart geneigt in die Scheibe 3 eingefügt, dass die Mittenachsen von durch benachbarte Gräben 11 definierten Kanälen auf eine gemeinsame virtuelle Punktquelle weisen. Dies gilt entsprechend auch für die Mittenachsen der Vertiefungen 15. Demgemäß ist in einer Ausführungsform der Erfindung vorgesehen, dass die Mittenachsen 17 der Vertiefungen 15 auf eine gemeinsame virtuelle Punktquelle gerichtet sind. Eine solche Anordnung zeigt schematisch Fig. 5. Die Mittenachsen 17 der Vertiefungen 15 koinzidieren im Allgemeinen auch mit Mittenachsen der durch die benachbarten Gräben 11 definierten Kanäle 12. In den Kanälen 12 ist zusätzlich zu den Vertiefungen 15 noch das Material der Scheibe 3 enthalten, welches die Wandungen 16, 19 bildet. Wie aus der Darstellung ersichtlich, sind auch die Gräben 11 auf die virtuelle Punktquelle 18 gerichtet, so dass die Wandungen der Gräben 11 in Richtung auf die Punktquelle 18 verlaufen. Durch den konischen Strahlengang würden sich wie in der Darstellung auch die Breiten der Vertiefungen 15 und Gräben 11 im Verlauf innerhalb der Scheibe 3 ändern. Dabei wären die Breiten auf der der Punktquelle 18 zugewandten Seite (hier die zweite Seite 7) geringer als auf der gegenüberliegenden Seite. Dies ist in der Praxis aber in der Regel nicht notwendig, da die Punktquelle im Allgemeinen im Verhältnis zur Dicke der Scheibe 3 wesentlich weiter entfernt ist, als in der Darstellung der Fig. 5. Bevorzugt ist es daher, dass die Gräben 11 und Vertiefungen 15 eine gleichbleibende Breite in Strahlrichtung, beziehungsweise in Richtung senkrecht zu den Seiten 5, 7 aufweisen. Dieser Verlauf der Breiten entspricht demgemäß der Darstellung der Fig. 2.

Nachfolgend wird ein Verfahren zur Herstellung eines Abschirmgitters 1 gemäß der Erfindung beschrieben. Das Verfahren zur Herstellung eines Abschirmgitters 1 gegen Röntgen-Streustrahlung umfasst die Schritte:

- Bereitstellen einer Scheibe 3 mit einer ersten Seite 5 und einer der ersten Seite 5 gegenüberliegenden zweiten Seite 7, und
- Bestrahlen der Scheibe 3 mit einem Laserstrahl, wobei das Material der Scheibe 3 für den Laserstrahl transparent ist, so dass der Laserstrahl in die Scheibe 3 eindringt, wobei
- der Laserstrahl entlang seines Pfades durch die Scheibe 3 filamentförmige Schädigungen hinterlässt, und wobei die filamentförmigen Schädigungen so eingefügt werden, dass eine erste Gruppe der filamentförmigen Schädigungen an der ersten Seite und eine zweite Gruppe der filamentförmigen Schädigungen an der zweiten Seite 7 endet,
- Entfernen des Materials der Scheibe 3 im Bereich der ersten und zweiten Gruppen von filamentförmigen Schädigungen durch Ätzen der Scheibe 3 mit einem Ätzmedium, so dass
- durch das Entfernen des Materials im Bereich der zweiten Gruppe eine Anordnung von Vertiefungen 15, welche zur zweiten Seite 7 der Scheibe 3 hin geöffnet sind, und
- durch das Entfernen des Materials im Bereich der ersten Gruppe ein Gitter 9 aus zur ersten Seite 5 hin geöffneten Gräben 11 erzeugt wird, und wobei dann
- in die Gräben 11 ein Röntgenstrahlungs- absorbierendes Material 13 gefüllt wird.

Um die filamentförmigen Schädigungen einzufügen, ist insbesondere ein Ultrakurzpulslaser geeignet. Fig. 6 zeigt dazu eine Laser-Bearbeitungsvorrichtung zum Einfügen von filamentförmigen Schädigungen 41 in die Scheibe 3, um nachfolgend hieraus in einem Ätzprozess die Gräben 11 und Vertiefungen 15 zu erzeugen. Die Vorrichtung 50 umfasst einen Ultrakurzpulslaser 51 mit vorgeschalteter Fokussierungsoptik 52 und eine Positioniereinrichtung 53. Mit der Positioniereinrichtung 53 kann der Auftreffpunkt 54 des Laserstrahls 40 des Ultrakurzpulslasers 51 auf einer der Seiten 5,7 einer zu bearbeitenden Scheibe 3 lateral positioniert werden. Bei dem dargestellten Beispiel umfasst die Positioniereinrichtung 53 einen x-y-Tisch, auf dem die Scheibe 3 auf einer ihrer Seiten 5, 7 aufliegt. Alternativ oder zusätzlich möglich ist aber auch, die Optik beweglich auszubilden, um den Laserstrahl 40 zu bewegen, so dass der Auftreffpunkt 54 des Laserstrahls 40 über die Scheibe 3 bewegbar ist. Die Fokussierungsoptik 52 fokussiert den Laserstrahl 40 nun zu einem in Strahlrichtung, also dementsprechend quer zur bestrahlten Seite der Scheibe 3 langgezogenen Fokus. Ein solcher Fokus kann beispielsweise mit einer kegelförmigen Linse (ein sogenanntes Axikon) oder einer Linse mit großer sphärischer Aberration erzeugt werden. Die Steuerung der Positioniereinrichtung 53 und des Ultrakurzpulslasers 51 wird vorzugsweise mittels einer programmtechnisch eingerichteten Recheneinrichtung 55 durchgeführt. In der Darstellung der Fig. 6 trifft der Laserstrahl 40 senkrecht auf die Scheibe 3. Die Positioniereinrichtung 53 und/oder die Fokussierungsoptik 52 können aber gemäß einer bevorzugten Ausführungsform auch so ausgebildet sein, dass eine schräge Einstrahlung ermöglicht wird, um filamentförmige Schädigungen 41 zu erzeugen, welche jeweils in Längsrichtung parallel zu den vorgesehenen Mittenachsen 17, beziehungsweise allgemein parallel zur Richtung zur virtuellen Punktquelle 18 liegen. Allgemein, ohne Beschränkung auf hier dargestellte, spezielle Ausführungsbeispiele ist daher in Weiterbildung des Verfahrens vorgesehen, dass die filamentförmigen Schädigungen 41 zumindest teilweise schräg zu einer der Seiten 5, 7 der Scheibe 3 eingefügt werden. Indem mittels der Positionierungseinrichtung 53 sukzessive auf der Scheibe 3 nach einem vorbestimmten Muster verteilte Auftreffpunkte, beziehungsweise Positionen von filamentförmigen Schädigungen 41 eingefügt werden, lassen sich vorbestimmte Muster erzeugen, die dann in einem nachfolgenden Ätzschritt ausgearbeitet werden.

Gemäß einem Ausführungsbeispiel können folgende Parameter für den Laserstrahl verwendet werden: Die Wellenlänge des Laserstrahls beträgt 1064 nm, typisch für einen YAG-Laser. Es wird ein Laserstrahl mit einem Rohstrahldurchmesser von 12mm erzeugt, der dann mit einer Optik in Form einer Bikonvex-Linse mit einer Brennweite von 16mm fokussiert wird. Die Pulsdauer des Ultrakurzpulslasers beträgt weniger als 20 ps, in einem Ausführungsbeispiel etwa 10 ps. Die Pulse werden in Bursts mit 2 oder mehr, bevorzugt 4 oder mehr Pulsen abgegeben. Die Burstfrequenz beträgt 12-48 ns, in einem Beispiel etwa 20 ns, die Pulsenergie mindestens 200 Mikrojoule, die Burstenergie entsprechend mindestens 400 Mikrojoule. Der Ultrakurzpulslaser kann gemäß einer Ausführungsform mit einer Repetitionsrate für die Bursts, beziehungsweise Pulspakete betrieben werden, welche zwischen 1 kHz und 1000 kHz, vorzugsweise zwischen 2 kHz und 100 kHz, besonders bevorzugt zwischen 3 kHz und 200 kHz liegt. Diese Repetitionsrate und/oder die Scangeschwindigkeit können dabei so gewählt werden, dass ein gewünschter Abstand benachbarter Schädigungen/Kanälen erreicht wird. Andere Varianten des Nd:YAG-Lasers wie die durch Frequenzverdopplung (SHG) oder Frequenzverdreifachung (THG) erzeugten Wellenlängen 532 nm bzw. 355 nm oder auch ein bei einer Emissionswellenlänge von 1030 nm betriebener Yb:YAG-Laser können als Strahlquellen in geeigneter Weise verwendet werden.

Eine besondere Anpassung des Verfahrens für die Herstellung von Sacklöchern/Kanälen kann über die Strahlaufbereitung des Lasers erfolgen. Im Speziellen kann eine extrem kurzer Brennweite, insbesondere eine Brennweite von f<20mm verwendet werden. Gemäß einer alternativen oder zusätzlichen Ausführungsform wird ein stark aufgeweiteter Rohstrahl verwendet. Besonders bevorzugt weist dazu der Laserstrahl beim Auftreffen auf das Objektiv, beziehungsweise auf die Fokussierungsoptik einen Strahlradius von mindestens 4 mm auf.

Eine weitere Maßnahme ist eine Modifikation der Intensitätsverteilung. Dazu kann die maximale Intensität von der optischen Achse zu den Randbereichen der Fokussieroptik verlagert oder allgemein auf eine größere Fläche verteilt werden. Strahlprofile mit einem solchen Merkmal sind unter anderem ein Flat-top-Profil und ein Donut-Profil.

Da eine Ausrichtung der Vertiefungen und Gräben auf eine Punktquelle auch eine schräge Bestrahlung mit sich bringt, ist es weiterhin vorteilhaft, die Einkopplung insbesondere nahe dem Brewsterwinkel mit einer Orientierung der Polarisation parallel zur Substratoberfläche vorzunehmen. Weiterhin kann gegebenenfalls eine asymmetrische Beleuchtung der Fokussieroptik vorgenommen werden, um größere Inzidenzwinkel zu erreichen.

Ausführungsformen für das Verfahren des Einfügens filamentförmiger Schädigungen und die dafür geeigneten Laserparameter, sowie auch Parameter für das nachfolgende Ätzen können auch der DE 10 2017 101 673.2, der DE 10 2018 110 211.9 und der PCT/EP2021/077030 entnommen werden. Diese Anmeldungen werden bezüglich der Ausführungsformen für das Einfügen der filamentförmigen Schädigungen 41 und der Parameter für die Laserbestrahlung und das Ätzen vollumfänglich auch zum Gegenstand dieser Offenbarung gemacht.

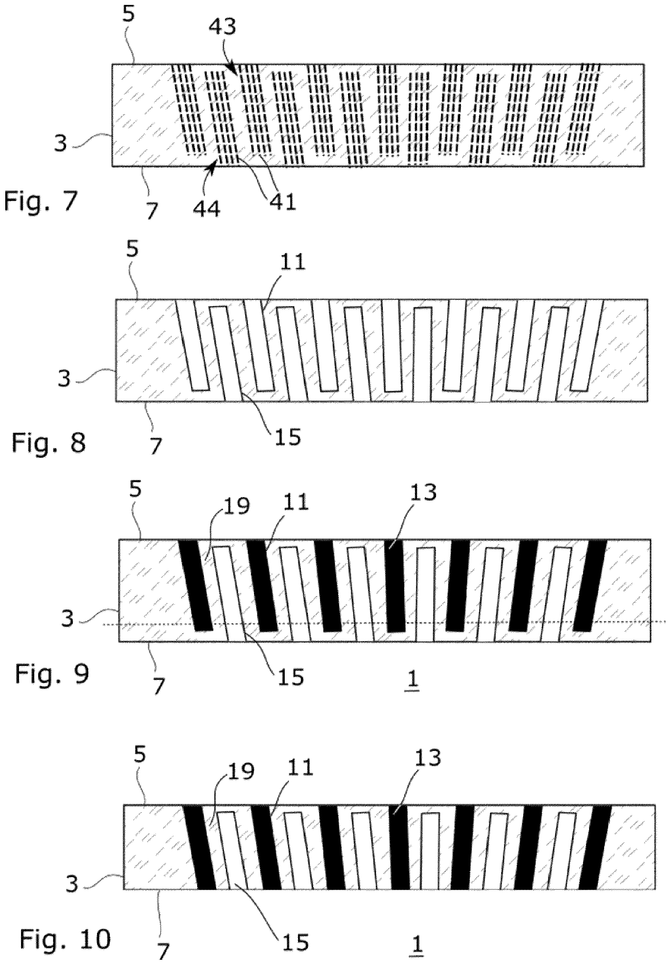

Fig. 7 zeigt eine Scheibe 3 mit eingefügten filamentförmigen Schädigungen. Die filamentförmigen Schädigungen 41 untergliedern sich in zwei Gruppen 43, 44. Dabei enden die filamentförmigen Schädigungen 41 der ersten Gruppe 43 an der ersten Seite 5 und die filamentförmigen Schädigungen 41 der zweiten Gruppe 44 an der zweiten Seite 7. Es ist ohne Beschränkung auf das dargestellte Beispiel allgemein besonders bevorzugt, wenn zumindest ein Teil, vorzugsweise alle der filamentförmigen Schädigungen 41, oder die filamentförmigen Schädigungen beider Gruppen 43, 44 wie dargestellt innerhalb der Scheibe 3 enden. Auf diese Weise werden beim nachfolgenden Ätzen in einfacher Weise Vertiefungen 15 in Form von Sacklöchern, beziehungsweise in der Scheibe endende Gräben 11 auch dann erzeugt, wenn die Scheibe allseitig dem Ätzmedium ausgesetzt wird. Die Filamente durchdringen vorzugsweise 50 % bis 90 % der Dicke des Glases, so dass nach dem Ätzvorgang die Kanäle oder Sacklöcher/Aushöhlungen, welche die Vertiefungen 15 und Gräben 11 bilden, vorzugsweise 55 % bis 95 % der Glasdicke erreichen.

Die filamentförmigen Schädigungen 41 enden damit also einerseits innerhalb der Scheibe, andererseits an einer der Seiten 5, 7. Wie ebenfalls anhand der Fig. 6 zu erkennen ist, sind die filamentförmigen Schädigungen 41 auch je nach Winkel der später vorgesehenen Richtung zur virtuellen Punktquelle schräg eingefügt. Es ist dabei gemäß einer Ausführungsform des Verfahrens nicht notwendig, die Scheibe 3 für das Einfügen der filamentförmigen Schädigungen 41 zu wenden. Vielmehr ist es ausreichend, die Lage des langgestreckten Fokus des Laserstrahls 40 anzupassen, beziehungsweise zu ändern. Dazu kann die Position der Linse und/oder die Position der Scheibe 3 geändert werden. Wird beispielsweise bei der Scheibe 3 gemäß Fig. 7 der Laserstrahl 40 auf die erste Seite 5 eingestrahlt, so kann der Beginn des langgestreckten Fokus für die Filamente der Gruppe 43 auf oder noch vor der Scheibe 3 liegen. Um die Filamente der Gruppe 44 einzufügen, kann dann der Fokus in Strahlrichtung so weit verschoben werden, dass der Beginn des langgestreckten Fokus des Laserstrahls 40 innerhalb der Scheibe 3 liegt. Ohne Beschränkung auf bestimmte Ausführungsbeispiele ist daher gemäß einer Ausführungsform des Verfahrens vorgesehen, dass zumindest ein Teil der filamentförmigen Schädigungen 41 beider Gruppen 43, 44 unter Einstrahlung des Laserstrahls 40 auf die gleiche Seite 5, 7 der Scheibe 3 und Änderung der Lage des Fokus des Laserstrahls 40 relativ zur Scheibe und in der Richtung entlang des Laserstrahls 40 erzeugt wird.

Wird als Material der Scheibe 3 ein Glas verwendet und als Ätzmedium eine Lauge, so kann besonders bei einer langsamen Ätzung eine charakteristische Oberflächentopographie auf den Wandungen der Gräben 11 und Vertiefungen 15 erzielt werden. Im Speziellen kann die Oberfläche der Gräben 11 und Vertiefungen eine Vielzahl aneinander angrenzender kleiner kalottenförmiger Vertiefungen aufweisen. Vorzugsweise weisen die kalottenförmigen Vertiefungen eine Tiefe auf, die kleiner ist als 10 $\mu$m, bevorzugt kleiner als 5 $\mu$m, bevorzugt kleiner als 2 $\mu$m, wobei die Tiefe durch eine Differenz zwischen einem Zentrum einer Vertiefungssenke und einem mittleren Gipfel des die Vertiefung umgebenden Grates definiert ist. Die kalottenförmigen Vertiefungen sind genauer auch in der DE 10 2017 101 673.2, der DE 10 2018 110 211.9 und der PCT/EP2021/077030 beschrieben, deren Inhalt diesbezüglich vollumfänglich auch zum Gegenstand dieser Offenbarung gemacht wird. Die Oberflächentopographie mit den kalottenförmigen Vertiefungen sorgt für eine gute Verbindung der Scheibe 3 mit dem röntgenabsorbierenden Material. Insbesondere kann eine feste Verbindung erreicht werden, wenn das röntgenabsorbierenden Material ein aufgeschmolzenes Glas, wie insbesondere ein Glaslot umfasst. Mit den Vertiefungen wird dabei die Oberfläche für einen Stoffschluss vergrößert und es wird eine regelrechte Verzahnung beider Materialien erreicht.

In Fig. 8 ist die Scheibe 3 nach dem Ätzen gezeigt. Das Material der Scheibe 3 ist entlang der filamentförmigen Schädigungen 41 wesentlich schneller ätzbar, als an Bereichen ohne solche Schädigungen. Daher wird das Material, vorzugsweise Glas beim Ätzen unter Erweiterung der filamentförmigen Schädigungen 41 entfernt. Die sich so bildenden Kanäle vereinigen sich schließlich und bilden die gewünschten Strukturen in Form von Gräben 11, die sich von der ersten Seite 5 ausgehend und Vertiefungen 15, die von der zweiten Seite 7 ausgehend in die Scheibe 3 hinein erstrecken. Entsprechend der Neigung der filamentförmigen Schädigungen 41 sind auch die Gräben 11 und Vertiefungen 15 zu einer virtuellen Punktquelle hin ausgerichtet, so wie in Fig. 5 dargestellt.

Schließlich kann auf der Seite 5 das Röntgenstrahlungs- absorbierende Material 13, beispielsweise in Form einer Paste in die Gräben 11 gefüllt werden, so dass das in Fig. 9 gezeigte Abschirmgitter 1 erhalten wird. Gemäß einer Weiterbildung des Verfahrens ist allgemein, ohne Beschränkung auf die dargestellte speziellen Ausführungsform vorgesehen, dass die Scheibe 3 nach dem Befüllen mit dem Röntgenstrahlungs- absorbierenden Material 13 auch gedünnt werden kann, beispielsweise um die Röntgenabsorbtion zu verringern. In Fig. 9 ist eine parallel und in der Nähe der zweiten Seite 7 verlaufende gestrichelte Linie eingezeichnet. Wird die Scheibe 3 nun auf der Seite 7 bis zu dieser Linie abgeschliffen, so wird eine Scheibe 3 erhalten, wie sie Fig. 10 zeigt. Gemäß noch einer alternativen oder zusätzlichen Weiterbildung, die auch in der Ausführungsform der Fig. 10 realisiert ist, werden beim Ausdünnen der Scheibe die Gräben 11 und damit das Röntgenstrahlungs- absorbierende Material 13 auf der zweiten Seite 7 freigelegt. Damit wird also allgemein eine Ausführungsform erhalten, bei welcher die Gräben 11 zumindest teilweise an beiden Seiten 5, 7 der Scheibe 3 offen sind. Diese Ausführungsform ist nicht auf das Ausdünnen beschränkt, beispielsweise besteht auch die Möglichkeit, die filamentförmigen Schädigungen 41 zumindest teilweise ganz durch die Scheibe 3 hindurch einzufügen, so dass die filamentförmigen Schädigungen 41 an beiden Seiten 5, 7 enden. Noch eine Möglichkeit ist, nahe beieinanderliegende filamentförmige Schädigungen 41 einzufügen, die abwechselnd auf der einen oder der anderen Seite 5, 7 enden. Beim Ätzen verbinden sich dann die sich entlang der filamentförmigen Schädigungen 41 ausbildenden Kanäle und es wird ein Graben 11 mit einer durchgehenden Verbindung zwischen beiden Seiten 5, 7 geschaffen. Hier besteht allerdings unter Umständen keine direkte Verbindung mehr zwischen den an einen Graben 11 angrenzenden Abschnitten der Scheibe 3. Um die mechanische Stabilität aufrechtzuerhalten, können aber beispielsweise Stege stehen gelassen werden, die durch Bereiche der Gräben 11 mit reduzierter Tiefe erhalten werden. Eine alternative oder zusätzliche Möglichkeit ist, ein Röntgenstrahlungs-absorbierendes Material vorzusehen, welches einen Stoffschluss mit der Wandung des Grabens 11 macht. Allgemein, nicht nur für die Variante der Fig. 10, ist es bevorzugt, ein Glas, insbesondere ein Glaslot als Bestandteil des Röntgenstrahlungs-absorbierenden Materials 13 vorzusehen, wobei das Glas aufgeschmolzen wird und so einen Stoffschluss mit dem Material der Scheibe 3, vorzugsweise ebenfalls ein Glas, herstellt. Gegebenenfalls reicht ein Anschmelzen des Glases aus. Geeignet als Bestandteil des Röntgenstrahlungs-absorbierenden Materials 13 ist insbesondere ein Blei- und/oder Bismut-haltiges Glas, wie etwa ein Bleiglas, unabhängig davon, ob es in aufgeschmolzener Form vorliegt oder nicht. Blei- und/oder Bismut-haltiges Glas hat aufgrund seines Blei-, beziehungsweise Bismut-Gehalts eine gute Abschirmwirkung gegenüber ionisierender Strahlung, insbesondere gegenüber Röntgenstrahlung. Das Glas kann auch ganz oder teilweise kristallisieren. So sind speziell kristallisierende Gläser bzw. Glaslote bekannt. Auch in diesem Fall wird im Sinne dieser Offenbarung aber von einem auf- oder angeschmolzenen Glas als Bestandteil des Röntgenstrahlungs-absorbierenden Materials gesprochen. Allgemein ist demgemäß in einer Ausführungsform vorgesehen, dass das Röntgenstrahlungs- absorbierende Material 13 ein an- oder aufgeschmolzenes Glas umfasst.

Weiterhin ist es für einen Stoffschluss von röntgenabsorbierendem Material 13 und Scheibe 3 und/oder eine dauerhafte, stabile Fixierung des Röntgenstrahlungs- absorbierenden Materials 13 in den Gräben 11 von Vorteil, wenn das Röntgenstrahlungs- absorbierende Material 13 ein aufgeschmolzenes Glaslot umfasst. Sehr vorteilhaft ist hier, dass es bleihaltige Glaslote gibt. Gerade Bleioxid als Glaskomponente ist geeignet, einen niedrigen Erweichungspunkt zu bewirken und stellt gleichzeitig eine hohe Abschirmwirkung bereit. Damit das Glas in den Gräben 11 aufgeschmolzen werden kann, ohne dass sich die Scheibe 3 deformiert, ist es allgemein bevorzugt, wenn für das Glas des Röntgenstrahlungs- absorbierenden Materials 13 und das Glas der Scheibe 3 zumindest eine der folgenden Merkmale gilt:

- das Glas des Röntgenstrahlungs- absorbierenden Materials 13 hat eine Glasübergangstemperatur $T_g$, die mindestens 100 °C, vorzugsweise mindestens 160 °C oder sogar mindestens 220 °C niedriger ist als die Glasübergangstemperatur $T_g$ des Glases der Scheibe 3,
- das Glas des Röntgenstrahlungs-absorbierenden Materials 13 hat eine Verarbeitungstemperatur, bei der dessen Viskosität bei $10^4$ dPa•s ist, die um mindestens 100 °C, insbesondere mindestens 200 °C, besonders bevorzugt mindestens 250 °C niedriger liegt, als die Verarbeitungstemperatur des Glases der Scheibe 3,
- das Glas des Röntgenstrahlungs-absorbierenden Materials 13 hat eine Verarbeitungstemperatur, bei der dessen Viskosität bei $10^4$ dPa•s ist, die niedriger ist als die Temperatur des Erweichungspunkts des Glases der Scheibe 3, wobei der Erweichungspunkt durch eine Viskosität von $10^{7,6}$ dPa•s definiert ist.

In einem Beispiel wird für das Röntgenstrahlungs-absorbierende Material 13 ein Glaslot des Typs G017-52 der Schott-AG verwendet. Bei einer Scheibe 3 aus dem Glas Borofloat 33 der Schott-AG ergibt sich ein Unterschied der Glasübergangstemperaturen Tg von 263 °C. Die Viskosität des Glaslots G017-052 beträgt $10^{7,6}$ dPas bei 347 °C. Diese Temperatur liegt um 218°C niedriger als der Erweichungspunkt des Glases Borofloat 33.

Ein Glas als Bestandteil des Röntgenstrahlungs-absorbierenden Materials, unabhängig davon, ob das Glas Blei enthält oder nicht, weist noch einen weiteren Vorteil in Verbindung mit der hier beschriebenen Anordnung auf. Glas kann als sprödbrüchiger Werkstoff sehr fein gemahlen werden. Der Glasstaub ist dann auch bestens, beispielsweise als Bestandteil einer pastösen Zubereitung geeignet, in die schmalen, tiefen Gräben 11 eingefüllt zu werden. Das Einfüllen kann dann in einfacher Weise beispielsweise durch Rakeln erfolgen. In einem Ausführungsbeispiel wird die pastöse Zubereitung in die Gräben 11 gefüllt, dann vorzugsweise bei 100 °C bis 200 °C getrocknet. Das Einfüllen und Trocknen kann insbesondere bei einem großen Aspektverhältnis der Gräben 11 wiederholt werden, um die Gräben 11 vollständig zu füllen. Sind die Gräben 11 hinreichend gefüllt, kann ein Tempern bei 350 °C bis 450 °C erfolgen, bei dem das Glas der Zubereitung aufgeschmolzen wird, um ein fest mit den Wandungen der Gräben 11 verbundenes Röntgenstrahlungs- absorbierendes Material 13 zu erhalten. Gemäß einer Ausführungsform ist daher vorgesehen, dass ein pulverförmiges Glas, vorzugsweise ein Glaslot 21 als Bestandteil des Röntgenstrahlungs- absorbierenden Materials 13, vorzugsweise eine Mischung eines pulverförmigen Glases mit Partikeln in die Gräben 11 eingefüllt wird, und wobei das Glaslot 21 auf- oder angeschmolzen wird, so dass ein festes Röntgenstrahlungs-absorbierendes Material 13 erzeugt wird, welches an den Wänden der Gräben 11 anhaftet.

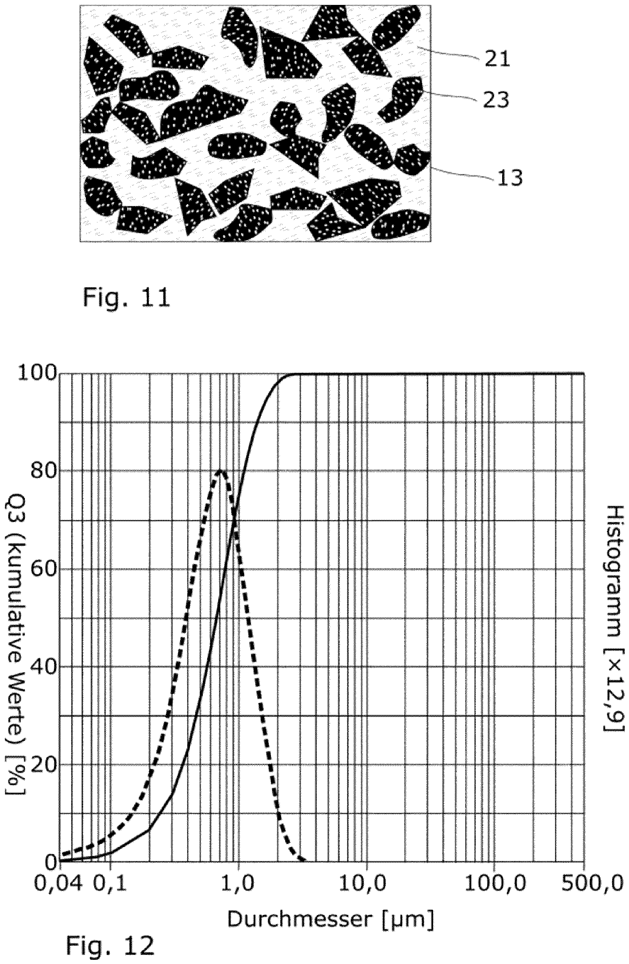

Fig. 11 zeigt schematisch eine bevorzugte Ausführungsform des Röntgenstrahlungs-absorbierenden Materials. Das Röntgenstrahlungs- absorbierende Material 13 enthält gemäß dieser Ausführungsform allgemein Partikel, vorzugsweise Metallpartikel 23. In besonders bevorzuger Weiterbildung umfasst das Röntgenstrahlungs- absorbierende Material 13 in einem aufgeschmolzenen Material eingebettete Partikel, insbesondere Metallpartikel 23 und/oder mineralische Partikel und/oder keramische Partikel. Unter einer Komponente in Partikelform oder als Partikel werden im Sinne dieser Offenbarung neben metallischen Körnern, beziehungsweise Partikeln auch keramische oder glaskeramische bzw. teilkristalline Gläser sowie auch Kristallite (ein oder polykristallin) als Körner, beziehungsweise Partikel, oder eine Kombination daraus verstanden. Bei dem aufgeschmolzenen Material kann es sich bevorzugt wie im dargestellten Beispiel um ein aufgeschmolzenes Glas oder Glaslot 21 handeln. Das Glas sorgt für eine feste und im Allgemeinen auch mechanisch belastbare Verbindung des Röntgenstrahlungs-absorbierenden Materials 13 mit der Scheibe 3. Ohne Beschränkung auf spezielle Ausführungsformen ist daher in einer Weiterbildung des Herstellungsverfahrens vorgesehen, dass eine Paste bereitgestellt wird, welche Glaspartikel und Partikel, vorzugsweise Metallpartikel 23 enthält, wobei die Paste vorzugsweise durch Rakeln in die Gräben 11 gefüllt wird, und wobei die Scheibe 3 mit den Glaspartikeln und Partikeln in den Gräben 11 aufgeheizt wird, so dass die Glaspartikel erweichen und ein Röntgenstrahlungs- absorbierendes Material 13 mit in Glas eingebetteten Partikeln, vorzugsweise Metallpartikeln 23 erhalten wird.

Ohne Beschränkung auf spezielle Ausführungsbeispiele ist gemäß einer bevorzugten Ausführungsform vorgesehen, dass die Paste zusätzlich zu den jeweiligen Feststoffen, vorzugsweise mindestens Glasstaub und Metallstaub, beziehungsweise Metallpartikel 23 organische Lösungs- oder Suspensionsmittel enthält. Allgemein ist die Paste in bevorzugter Ausgestaltung also ein Gemisch des gemahlenen Glases mit Metallstaub und einem oder mehreren organischen Lösungsmitteln. Gegebenenfalls sind weitere Zuschlagstoffe, beispielsweise kristalline anorganische Materialien enthalten. Bevorzugt als organische Lösungs- oder Suspensionsmittel sind flüssige organische Stoffe mit niedriger Viskosität und hohem Siedepunkt. Vorzugsweise beträgt der Siedepunkt mindestens 120 °C, besonders bevorzugt mindestens 180 °C. Die Viskosität ist bei 20°C vorzugsweise kleiner als 5 mPa•s. Besonders geeignet sind verschiedene Glykolether.

Für die Metallpartikel 23 ist es sinnvoll, ein besonders schweres Metall oder eine Legierung mit mindestens einem schweren Metall zu verwenden. Generell ist es bevorzugt, dass die Metallpartikel 23 Metalle mit einer Ordnungszahl größer als 55 zu einem Anteil von mindestens 66 at% enthalten. Gemäß einer alternativen oder zusätzlichen Ausführungsform weisen die Metallpartikel 23 eine Dichte von mindestens 9 g/cm$^3$ auf, um eine gute Röntgenabsorbtion zu erzielen. Insbesondere ist die Dichte des Röntgenstrahlungs-absorbierenden Materials 13 bevorzugt wesentlich höher als die Dichte der Scheibe 3. Damit wird eine sehr unterschiedliche Röntgenabsorbtion in den verschiedenen Materialien und entsprechend ein hoher Kontrast erzielt. Daher ist es bevorzugt, wenn die Dichte des Röntgenstrahlungs-absorbierenden Materials 13 mindestens um einen Faktor vier größer ist, als die Dichte des Materials der Scheibe 3.

Besonders bevorzugt umfasst das Röntgenstrahlungs-absorbierende Material 13 Partikel eines Wolfram enthaltenden Materials, vorzugsweise in Form von metallischem Wolfram oder einer Wolfram-Legierung. Wolfram weist eine besonders hohe Dichte auf. In reiner metallischer Form hat Wolfram eine Dichte von 19,25 g/cm$^3$. Alternativ oder zusätzlich können auch Partikel eines Wolfram haltigen Minerals und/oder einer Wolfram-haltigen Keramik enthalten sein. Mit schweren Metallen, wie etwa Wolfram kann auch eine hohe Dichte des Röntgenstrahlungs- absorbierenden Materials 13 erreicht werden. So ist in einer bevorzugten Ausführungsform vorgesehen, dass das Röntgenstrahlungs-absorbierende Material 13 eine Dichte von mindestens 9 g/cm$^3$, vorzugsweise mindestens 11 g/cm$^3$ aufweist. Besonders mit einer Kombination aus Metallpartikeln schwerer Metalle und Glas, insbesondere Bleiglas oder einem Glaslot lassen sich solche hohen Dichten erzielen. Diese Dichten sind auch für Röntgenstrahlungs- absorbierende Materialien nicht selbstverständlich. So erreichen mit Wolfram versehene Kunststoffe oder Polymerpasten typischerweise nur Dichten etwas oberhalb von 8 $g/cm^3$.

Durch die Inhaltsstoffe des Röntgenstrahlungs- absorbierenden Materials 13 kann weiterhin auch dessen Temperaturausdehnungskoeffizient eingestellt werden. Durch Wahl der Inhaltsstoffe und eines geeigneten Materials der Scheibe 3, insbesondere eines geeigneten Glases kann gemäß noch einer Weiterbildung des Abschirmgitters 1 die Differenz der linearen Temperaturausdehnungskoeffizienten von Röntgenstrahlungs-absorbierendem Material 13 und dem Material der Scheibe 3 auf im Betrag kleiner als 3 ppm/K begrenzt werden. Um die Ausdehnungskoeffizienten aneinander anzupassen oder die Differenz der Ausdehnungskoeffizienten von Scheibe und Röntgenstrahlungs-absorbierendem Material zumindest zu verringern, ist gemäß einer Ausführungsform vorgesehen, im Röntgenstrahlungs-absorbierenden Material zumindest eine Komponente, vorzugsweise in Partikelform, beziehungsweise als partikelförmigen Zuschlagstoff für dessen Zubereitung vorzusehen, welche in zumindest einem Teil des Temperaturbereichs zwischen 0 °C und 200 °C einen linearen Temperaturausdehnungskoeffizienten aufweist, der kleiner als 1 ppm/K oder sogar negativ ist. Die Partikel können beispielsweise zumindest teilweise in Form von Kristalliten vorliegen. Besonders vorteilhaft ist hier, dass es auch Materialien mit hoher Dichte gibt, die diese Bedingungen erfüllen und daher auch noch eine gute Abschirmwirkung aufweisen. Im Speziellen sind Blei- oder Wolfram-Verbindungen bekannt, die solche Temperaturausdehnungskoeffizienten aufweisen. In Weiterbildung dieser Ausführungsform enthält das Röntgenstrahlungs-absorbierende Material 13 dabei zumindest eine der Verbindungen Bleititanat und/oder Zirkonwolframat.

Gemäß noch einer weiteren Ausführungsform sind auch die linearen thermischen Ausdehnungskoeffizienten des Glases des Röntgenstrahlungs-absorbierenden Materials 13 und der Metallpartikel 23 aufeinander angepasst. Vorzugsweise werden das Glas und die Metallpartikel 23 so ausgewählt, dass die betragsmäßige Differenz der linearen thermischen Ausdehnungskoeffizienten höchstens 5 ppm/K beträgt. Nachfolgend wird ein Ausführungsbeispiel für das Röntgenstrahlungs-absorbierende Material 13, beziehungsweise für die Inhaltsstoffe einer Paste zur Herstellung des Röntgenstrahlungs-absorbierende Material 13 beschrieben. Als Glas kommt ein Bleioxid-haltiges Glaslot des Typs G017-52 zum Einsatz. Das Glas ist hoch bleihaltig, mit einem Gehalt von PbO von 86 Gewichtsprozent. Als weitere Komponente kommen Wolfram-Metallpartikel hinzu. Die Eigenschaften und die Zusammensetzung des daraus hergestellten Röntgenstrahlungs- absorbierenden Materials sind in der nachfolgenden Tabelle angegeben:

| Tabelle: | |
|---|---|
| Linearer thermischer Ausdehnungskoeffizient Wolfram | $4{,}5 \cdot 10^{-6}$ $K^{-1}$ |
| Dichte Wolfram | 19,25 $g/cm^3$ |
| Linearer thermischer Ausdehnungskoeffizient Glas | $11{,}4 \cdot 10^{-6}$ $K^{-1}$ |
| Dichte Glas | 6,65 $g/cm^3$ |
| Materialkombination: | |
| Volumenanteil Wolfram | 60,85% |
| Massenanteil Wolfram | 81,82% |
| Volumenanteil Glas | 39,15% |
| Massenanteil Glas | 18,18% |
| Ausdehnungskoeffizient der Kombination | $7{,}2 \cdot 10^{-6}$ $K^{-1}$ |
| Dichte der Kombination: | 14,34 $g/cm^3$ |

Wie anhand dieses Ausführungsbeispiels ersichtlich kann durch die Kombination der Materialien eine sehr hohe Dichte von deutlich über 9 $g/cm^3$, sogar noch deutlich über 11 $g/cm^3$ erreicht werden.

Ein weiteres Glas, welches für das Röntgenstrahlungs-absorbierende Material verwendet werden kann, ist das Bismut-Glaslot G018-423 der Schott AG. Dieses Glaslot enthält bis zu 84 Gewichtsprozent $Bi_2O_3$.

Eine Herausforderung beim oben beschriebenen Befüllen der Gräben 11 durch Rakeln ist, dass die Gräben 11 aufgrund des hohen Aspektverhältnisses sehr schmal und tief sind. Die Gräben 11 sollten dennoch möglichst vollständig werden. Auch sollten sich keine oder möglichst wenige und kleine Poren sich nach dem Aufschmelzen des Glases bilden. Diese Eigenschaften lassen sich überraschend wirksam durch die Korngrößen der Materialien beeinflussen.

Fig. 12 zeigt eine Partikelgrößenverteilung für ein mit einem Attritor gemahlenes Glaslot. Ein geeigneter Glastyp ist unter anderem das Lotglas G017-052 der Anmelderin. Die gestrichelte Kurve ist das Histogramm der Glaspartikel-Durchmesser. Die durchgezogene Linie kennzeichnet den kumulativen Verlauf der Partikelgrößen. Die Glaspartikel sind nicht kugelförmig, der auf der Abszisse des Diagramms angegebene Durchmesser bezeichnet daher den Mittelwert der lateralen Abmessungen. Das Glaspulver weist einen 90%-Durchmesser von 1,39 μm auf. Dies ist der Durchmesser, den 90% der Glaspartikel höchstens haben. Der 50%-Durchmesser liegt bei 0,67 μm und der 10%-Durchmesser bei 0,24 μm. Gemäß einer Weiterbildung des Verfahrens ist nun vorgesehen, die verwendeten Komponenten, also insbesondere das Glaspulver und die Metallpartikel so auszuwählen, dass der Füllgrad maximiert wird. Typischerweise wird dies unter der Nebenbedingung vorgenommen, dass der Gehalt an Metallpartikeln möglichst hoch ist. Auch unter dieser Nebenbedingung und eventuell vorgegebenen Partikelgrößenverteilungen kann der Füllgrad angepasst werden, indem der Paste, beziehungsweise allgemeiner der Mischung der Glaspartikel und Metallpartikel zwei Metallpulver mit unterschiedlichen Partikelgrößenverteilungen zugegeben werden. Durch unterschiedliche Wichtungen beider Pulver und unter Berücksichtigung der Partikelgrößenverteilung des Glaspulvers kann dann der Füllgrad optimiert werden. Gemäß einer bevorzugten Ausführungsform beträgt der Füllgrad des eingefüllten und gegebenenfalls, vorzugsweise wie oben beschrieben durch Auf- oder Anschmelzen eines Glases verfestigten Materials 13 in den Gräben 11 mindestens 40 Vol%, vorzugsweise mindestens 60 Vol%. Angestrebt und erreichbar ist sogar ein Füllgrad von mindestens 70 Vol%.

Der Füllgrad kann beispielsweise mit einem Andreasen-Modell berechnet werden. Bei einem Andreasen-Modell wird eine Partikelgrößenverteilung angenommen, welche die Form

$$F(d) = 100 * \left(\frac{d}{d_{max}}\right)^n$$

Die vom Partikeldurchmesser abhängigen Funktionswerte F(d) sind dabei die kumulativen Prozentsätze der Partikel mit einem Durchmesser kleiner oder gleich d. Die Funktionswerte F(d) entsprechen daher den kumulativen Werten Q3, beziehungsweise der durchgezogenen Linie des Diagramms der Fig. 12. Die Form der Verteilung wird insbesondere durch die Konstante n bestimmt. Der Parameter $d_{max}$ gibt den maximalen Durchmesser der vorhandenen Partikel an.

Eine Optimierung kann auch mit anderen Modellen, die dem Fachmann bekannt sind, durchgeführt werden. Alternative Modelle sind beispielsweise das Ψ-Modell, oder das Dinger-Funk-Modell.

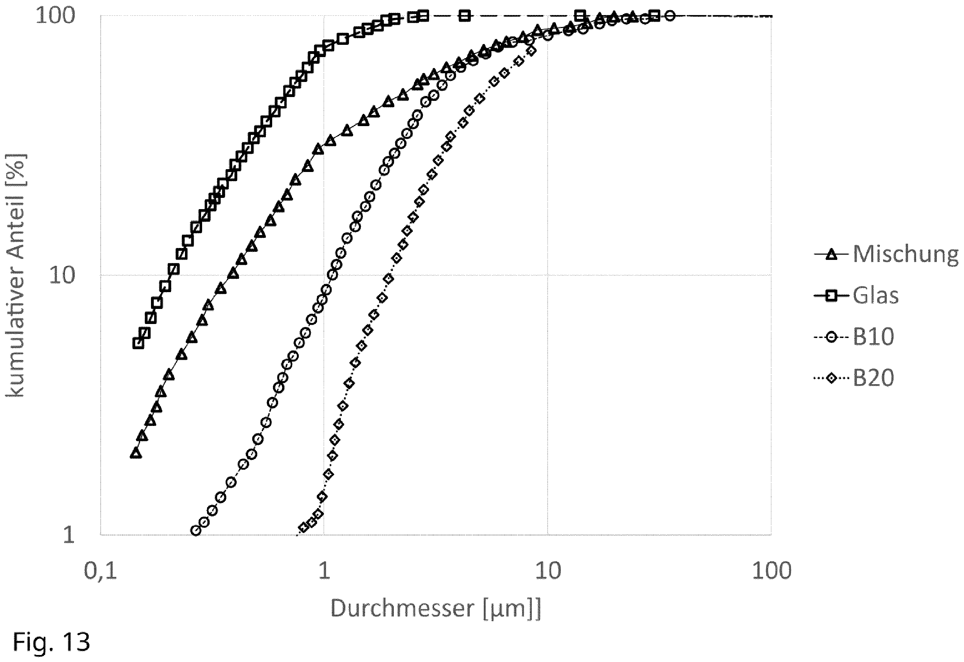

Fig. 13 zeigt Partikelgrößenverteilungen gemäß einem Ausführungsbeispiel. Im Speziellen sind zusätzlich zur Partikelgrößenverteilung des gemahlenen Glases aus Fig. 12 noch zwei Partikelgrößenverteilungen für Wolfram- Metallpulver B10 und B20, sowie der Mischung aller drei Komponenten, also des gemahlenen Glases mit den beiden Metallpulvern gezeigt. Die Partikelgrößenverteilungen können unter anderem anhand ihres d50-Werts charakterisiert werden. Dies ist der Wert, bei welchem die Anzahl größerer und kleinerer Partikel gleich ist. Mit anderen Worten, die Hälfte aller Partikel des jeweiligen Staubs oder Mahlguts hat einen Durchmesser kleiner als der d50-Wert. Der d50-Wert des Metallpulvers B10 beträgt 3 μm, der d50-Wert des Metallpulvers B20 liegt bei 5 μm und der d50-Wert des gemahlenen Glases bei nur 0,67 μm. Die Metallpulver B10 und B20 werden von der Firma A.L.M.T. Corp. (JP) angeboten.

Wie anhand von Fig. 13 ersichtlich weist das Glas die kleinsten und das Metallpulver B20 die größten Partikelgrößen auf. Gemäß einer Ausführungsform werden also die zumindest drei Komponenten, nämlich hier das gemahlene Glas und die beiden Metallpulver so gemischt, dass eine möglichst hohe Packungsdichte erhalten wird. Das Verfahren beinhaltet also gemäß einer Weiterbildung allgemein die Schritte des Messens oder Berechnens einer Packungsdichte einer Mischung eines gemahlenen Glases (oder Glasstaubs) mit zumindest einem weiteren Pulver, Berechnen und/oder auch Messen der Packungsdichte der Mischung, zumindest einmaliges Berechnen oder Messen der Packungsdichte einer Mischung mit variiertem Mischungsverhältnis, aus den zumindest zwei Ergebnissen der Berechnung oder Messung Auswählen oder Bestimmen eines Mischungsverhältnisses für eine Zubereitung zum Befüllen der Gräben 11 und Herstellung einer Zubereitung mit dem Mischungsverhältnis. Das ausgewählte Mischungsverhältnis für die Zubereitung kann, muss aber nicht mit einem der vermessenen oder berechneten Mischungsverhältnisse übereinstimmen. So kann ein ideales Mischungsverhältnis aus den Ergebnissen auch interpoliert oder extrapoliert werden.

Die Partikelgrößenverteilung einer hinsichtlich der Packungsdichte optimierten Mischung aus dem Glaspulver und den beiden Metallpulvern B10, B20 liegt mit seinen Partikelgrößen zwischen den Metallpulvern einerseits und dem Glasstaub andererseits. Gemäß dem Ausführungsbeispiel weist die Zubereitung folgende Mischung auf:

| Komponente | Gewichtsprozent | Volumenprozent | d50-Wert |
|---|---|---|---|
| Wolfram B10 | 21,82 | 16,19 | 3 μm |
| Wolfram B20 | 60 | 44,65 | 5 μm |
| Glas G017 -052 | 18,18 | 39,16 | 0,67 μm |

Die Wolfram-Metallpartikel haben also einen Gewichtsanteil von etwas über 80 Gewichtsprozent und einen Volumenanteil von etwa 30 Volumenprozent.

Alternativ oder zusätzlich zu Metallpartikeln können auch Minerale oder Keramiken eingesetzt werden, die schwere Elemente, insbesondere Schwermetalle enthalten. Unter einem schweren Element wird entsprechend der oben angegebenen Definition ein Element ab Ordnungszahl 55 verstanden. Auch hier gilt bevorzugt, dass das Röntgenstrahlungs-absorbierende Material ein Material ist, welches zu mindestens 10 Gewichtsprozent Elemente mit einer Kernladungszahl von mindestens $Z=56$, vorzugsweise zu mindestens 25 Gewichtsprozent, besonders bevorzugt zu einem Anteil von mindestens 50 Gewichtsprozent solche Elemente mit $Z \geq 56$ aufweist, auch wenn zusätzlich keine Metallpartikel enthalten sind. Geeignete Minerale sind unter anderem Scheelit, beziehungsweise Calzium-Wolframat ($CaWO_4$), Bleioxid und Bleisulfid.

Wird, wie bevorzugt die in die Gräben 11 eingefüllte Mischung erhitzt, so dass das Glas schmilzt oder erweicht und somit eine feste Matrix für die Metallpartikel 23 bildet, sind am fertiggestellten Abschirmgitter die Glaspartikel im Allgemeinen nicht mehr erkennbar. Charakteristisch für eine bevorzugte Mischung mit hoher Packungsdichte und damit auch für ein Röntgenstrahlungs- absorbierendes Material 13 mit hoher Absorptionswirkung ist aber auch, dass die Metallpartikel 23 aufgrund der Mischung zweier unterschiedlich feiner Metallstäube eine breite Größenverteilung aufweisen. Allgemein, ohne Beschränkung auf das oben erläuterte Beispiel ist daher ein Röntgenstrahlungs- absorbierendes Material 13 vorgesehen, bei welchem die vorzugsweise in Glas fixierten Partikel in Form einer Mischung mindestens zweier Pulver, insbesondere Metallpulver, und/ oder Mineralpulver und/oder Keramikpulver mit unterschiedlichen d50-Werten vorliegen. Insbesondere können sich diese d50-Werte auch, wie im Ausführungsbeispiel, um mindestens 1,5 μm unterscheiden. Einhergehend mit dieser Mischung geht auch, dass die Partikelgrößenverteilung vergleichsweise breit ist, zumindest deutlich breiter als die Partikelgrößenverteilungen der beiden Metallstäube für sich genommen. Passt man eine Andreasen-Verteilung gemäß obiger Gleichung an eine solche reale Verteilung an, so ergibt sich ein vergleichsweise kleiner Exponent n. Gemäß einer weiteren Ausführungsform ist daher ein Röntgenstrahlungs- absorbierendes Material 13 vorgesehen, welches Partikel, vorzugsweise Metallpartikel 23 enthält, die eine Partikelgrößenverteilung aufweisen, für welche der Exponent (oder Modulus) n einer an die Partikelgrößenverteilung der Metallpartikel

$$F(d) = 100 * \left(\frac{d}{d_{max}}\right)^n$$

23 angepassten Andreasen-Verteilung der Partikeldurchmesser d, kleiner als $n=0,33$, vorzugsweise kleiner als $n=0,28$ ist, wobei $d_{max}$ der maximale Partikeldurchmesser der angepassten Andreasen-Verteilung ist. Da die Andreasen-Verteilung idealisiert und an die tatsächliche Verteilung der Metallpartikel 23 angepasst ist, muss der $d_{max}$-Wert nicht mit einem tatsächlichen maximalen Partikeldurchmesser der Metallpartikel 23 übereinstimmen. In der doppelt-logarithmischen Darstellung der Fig. 13 sind Andreasen-Verteilungen keine Kurven, wie die tatsächlichen gezeigten Partikelgrößenverteilungen, sondern Geraden.

Durch den Einsatz zweier unterschiedlich feiner Metallpulver kann die Partikelgrößenverteilung nicht nur breiter werden. Gemäß einer alternativen oder zusätzlichen Ausführungsform kann die Partikelgrößenverteilung der Partikel, insbesondere Metallpartikel 23 im Röntgenstrahlungs-absorbierenden Material 13 auch bi- oder multimodal sein. In der Darstellung der Partikelgrößenverteilung, wie sie für das gemahlene Glas in Fig. 12 gezeigt ist, bedeutet dies, dass das Histogramm zwei oder mehr unterscheidbare Maxima aufweist.

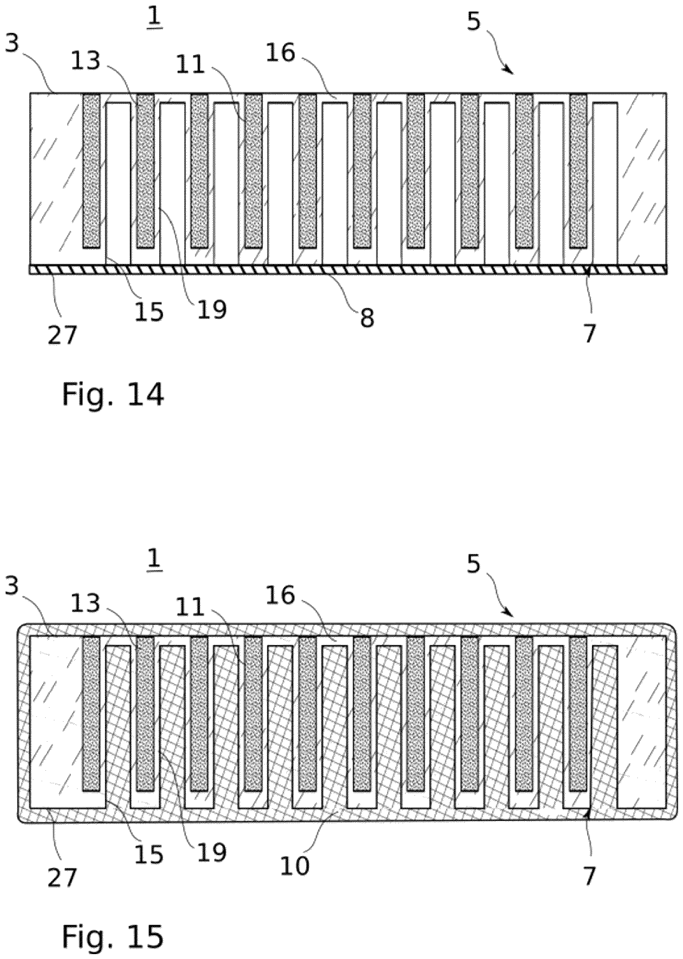

Neben den Wolfram-Stäuben können auch noch weitere Komponenten in Partikelform vorhanden sein. Gedacht ist hier beispielsweise an Zuschlagsstoffe, mit welchen sich der Temperaturausdehnungskoeffizient des Röntgenstrahlungs-absorbierenden Materials einstellen lässt, beispielsweise niedrigdehnende Stoffe oder sogar Komponenten mit negativer Temperaturausdehnung, wie Bleititanat oder Zirkonwolframat.Durch das Einfügen der Gräben 11 und Vertiefungen werden große Teile des Materials der Scheibe 3 entfernt. Insbesondere können die durchgehenden Materialverbindungen in der Scheibe 3 wie anhand der Ausführungsformen der Fig. 2, 8 und 9 ersichtlich ist, durch diese Strukturierung nicht geradlinig, sondern in einer Mäander verlaufen. Dies setzt die Stabilität der Scheibe 3 herab. Durch einen Materialschluss eines festen Röntgenstrahlungs-absorbierenden Materials 13, wie es etwa in Form des aufgeschmolzenen Glases, beziehungsweise Glaslots 2 vorgesehen ist, kann die Festigkeit wieder erhöht werden. Dennoch kann es wünschenswert sein, der strukturierten Scheibe 3 mehr Festigkeit zu verleihen. Eine Möglichkeit hierzu ist die Verbindung der Scheibe 3 mit einer weiteren Scheibe. Vorzugsweise kann dabei die Scheibe 3 mit einer Glasscheibe verbunden werden. Insbesondere kann hierzu eine Dünnglas-Scheibe verwendet werden. Fig. 14 zeigt dazu ein Beispiel dieser Ausführungsform. Gemäß einer Variante wird, wie dargestellt, die Dünnglas-Scheibe 8 auf der zweiten Seite 7 der Scheibe 3 befestigt. Ein Vorteil dieser Anordnung ist, dass zusätzlich die Vertiefungen 15 verschlossen werden, so dass eine Kontamination oder Verschmutzung der Vertiefungen 15 vermieden wird. Unter einer Dünnglas-Scheibe 8 wird im Sinne dieser Offenbarung eine Glasscheibe mit einer Dicke von höchstens 250 μm, vorzugsweise höchstens 150 μm verstanden. Ein Dünnglas ist in diesem Fall besonders geeignet, da die Röntgenabsorbtion aufgrund der geringen Materialstärke nur gering ist. Besonders vorteilhaft ist zugleich, dass selbst das dünne Glas die mechanische Stabilität der Anordnung erheblich erhöht. Dies liegt daran, dass die Vertiefungen 15 typischerweise nur eine geringe Breite, beziehungsweise laterale Breite aufweisen. Dadurch kann sich das Dünnglas über den Vertiefungen 15 kaum verbiegen, so dass eine große Zunahme der Steifigkeit erzielt wird. Gleiches gilt entsprechend auch für eine Dünnglas-Scheibe 8, die auf der ersten Seite 5 der Scheibe 3 befestigt ist. Allgemein, ohne Beschränkung auf das dargestellte Beispiel ist daher in einer Ausführungsform vorgesehen, dass auf wenigstens einer Seite 5, 7 der Scheibe 3 eine Dünnglas-Scheibe 8 befestigt ist. Zum Befestigen eignen sich mehrere Verfahren. Eine einfache Variante ist das Verkleben, beispielsweise mit einem Epoxidharz oder einem Silikon. Denkbar ist auch die Verwendung eines Glaslots, beispielsweise das gleiche Glaslot, welches auch für das Röntgenstrahlungs-absorbierende Material 13 verwendet wird. Die Verbindung mit einem Glaslot ermöglicht es, die Befestigung der Dünnglas-Scheibe 8 an der Scheibe 3 und das Aufschmelzen des Glaslots des Röntgenstrahlungs-absorbierenden Materials 13 in einem einzelnen Schritt durchzuführen. Ebenso ist es auch möglich, die beiden Scheiben 3, 8 mit einem Laser zu verschweißen. Auch ein anodisches Bonden ist denkbar.

Eine alternative oder zusätzliche Weiterverarbeitung des Abschirmgitters 1 ist das Aufbringen einer organischen Schicht oder Verkapselung, beispielsweise durch Tauchen. Ein Beispiel dazu zeigt Fig. 15. Bei diesem Beispiel ist die Scheibe 3 des Abschirmgitters 1 vollständig von einer organischen Verkapselung 10 umgeben. Die Verkapselung 10 kann auch wie dargestellt ganz oder wenigstens teilweise die Vertiefungen 15 ausfüllen. Sofern das organische Material keine hohe Röntgenabsorbtion aufweist, führt dies nicht zu erheblichen Absorbtionsverlusten innerhalb der Vertiefungen 15. Geeignet als organische Verkapselung sind insbesondere Polymere, Lacke, Kunstharze, Silikone. Anders als dargestellt kann die organische Verkaspelung 10 auch nur auf einem Teil der Scheibe 3, beispielsweise auf der ersten Seite 5 aufgebracht sein. Allgemein ist nach dieser Ausführungsform also vorgesehen, dass auf der Scheibe 3 zumindest teilweise, beziehungsweise auf zumindest einem Teil der Oberfläche der Scheibe 3 eine organische Verkapselung 10 aufgebracht ist. Diese Ausführungsform kann auch selbstverständlich mit der Ausführungsform mit der Dünnglas-Scheibe 8 kombiniert werden. Dabei kann die organische Verkapselung 10 beispielsweise als mechanischer Schutz für die Scheibe 3 und insbesondere die Dünnglas-Scheibe 8 dienen.

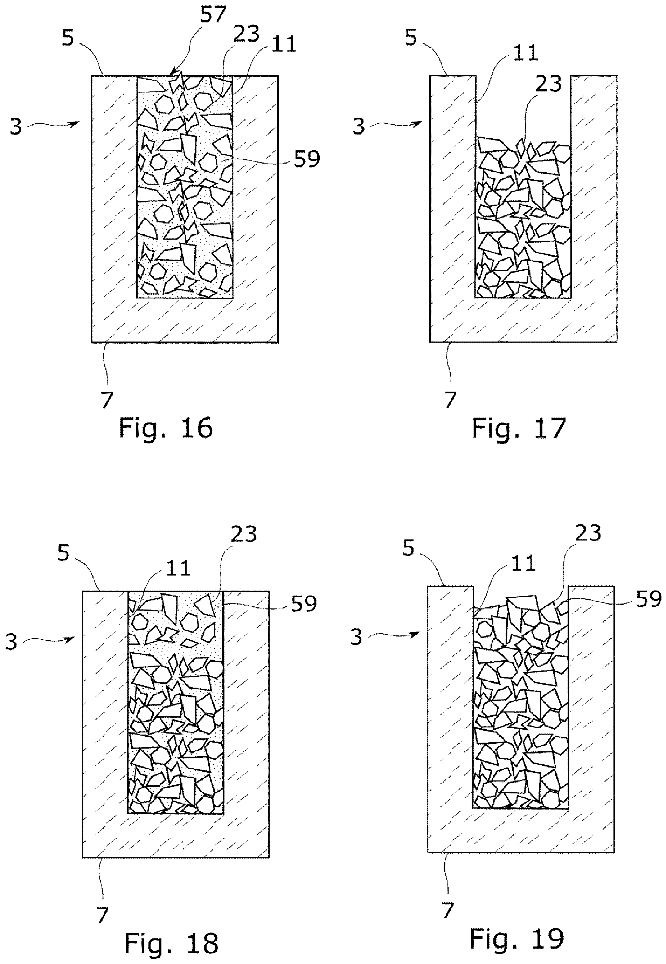

Im Folgenden werden verschiedene Ausführungsformen zur Befüllung der Gräben 11 der Scheibe 3 mit röntgenabsorbierendem Material beschrieben. Fig. 16 bis Fig. 19 zeigen Verfahrensschritte gemäß einer ersten Ausführungsform. Der Einfachheit halber ist nur ein Ausschnitt der Scheibe 3 mit einem einzelnen Graben 11 dargestellt. Um das Einfüllen zu erleichtern, ist es bevorzugt, die Kavitäten oder Gräben 11 zu evakuieren. In die Gräben 11 wird dann wie in Fig 16 dargestellt, eine Dispersion 57 mit den Metallpartikeln 23 und einem Dispergiermedium 59 eingefüllt. Die Dispersion 57 kann im Einklang mit den weiter oben beschriebenen Ausführungsformen generell auch als Paste bezeichnet werden. Die Dispersion kann neben den Metallpartikeln 23 wie bereits beschrieben auch andere Partikel, beispielsweise Glaspartikel enthalten.

Das Einfüllen erfolgt vorzugsweise unter Druck, um die Gräben 11 gut ausfüllen zu können. In einem nächsten Schritt wird die Dispersion 57 getrocknet, wobei das Dispersionsmedium 59 entfernt wird. Die Partikel setzen sich dadurch ab und verdichten sich. Dadurch kann es jedoch dazu kommen, dass, wie in Fig. 17 dargestellt die Gräben 11 nicht vollständig gefüllt sind. Daher kann der Prozess des Einfüllens der Dispersion, wie in Fig. 18 gezeigt, und das Trocknen, beziehungsweise das Entfernen des Dispersionsmediums, wie in Fig. 19 dargestellt, ein oder mehrmals wiederholt werden, um einen möglichst hohen Füllgrad zu erreichen. Im Idealfall wird dadurch, wie in Fig. 19 gezeigt, eine vollständige Füllung der Gräben 11 mit den Partikeln der Dispersion erreicht. Ohne Beschränkung auf die speziellen dargestellten Beispiele der Fig. 16 bis Fig. 19 ist also gemäß einer Ausführungsform ein Verfahren vorgesehen, bei welchem

- die Gräben 11 der vorzugsweise in einer Unterdruck-Umgebung, insbesondere im Vakuum gelagerten Scheibe 3 mit einer Dispersion 57 mit einem Dispergiermedium 59 und Metallpartikeln 23 befüllt werden,
- das Dispergiermedium 59 entfernt wird, so dass sich die Metallpartikel 23 absetzen, und
- diese beiden Schritte vorzugsweise mindestens einmal wiederholt werden.

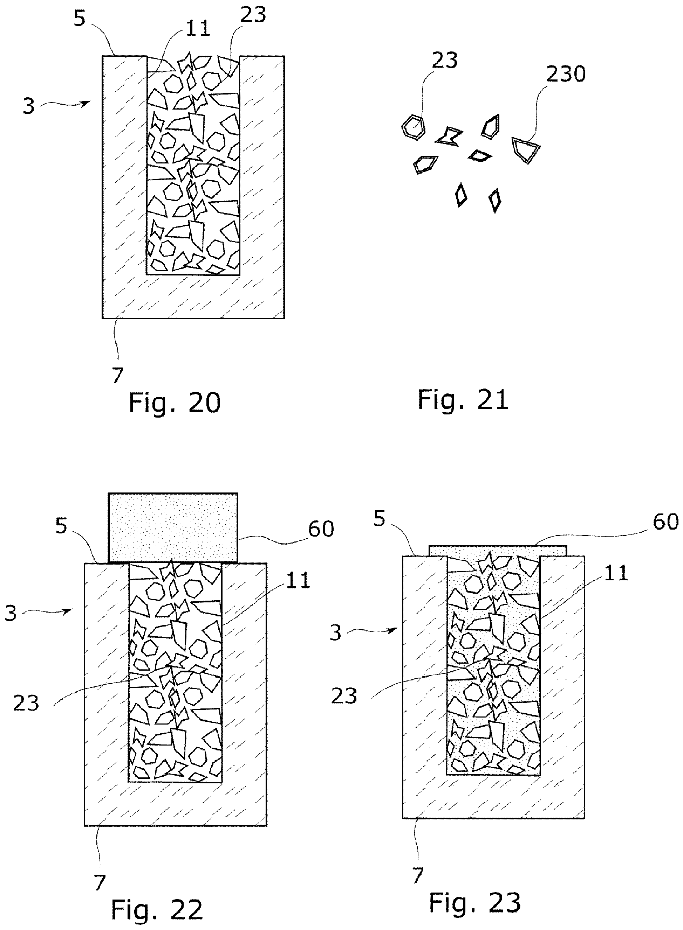

Gegebenenfalls können aber trotzdem noch Hohlräume in der Befüllung der Gräben 11 vorhanden sein. Alternativ oder zusätzlich zu einer möglichst hohen Verdichtung der Metallpartikel 23 in den Gräben 11 kann zur Lösung dieses Problems auch ein Verbund mit einem weiteren Metall, welches die Zwischenräume zwischen den Partikeln auffüllt, hergestellt werden. Die Fig. 20 bis Fig. 23 zeigen dazu Verfahrensschritte zur Befüllung gemäß einer weiteren Ausführungsform des Herstellungsverfahrens. Zunächst werden, wie in Fig. 20 dargestellt, die Gräben 11 mit Metallpartikeln 23 befüllt. Das Befüllen kann dabei auch entsprechend dem anhand der Fig. 16 bis Fig. 19 beschriebenen Verfahren erfolgen. Anschließend wird, wie in Fig. 22 dargestellt, ein Füllmetall 60 auf der Seite 5 der Scheibe 3 angeordnet. Dieses wird dann aufgeschmolzen, fließt in die Gräben 11 und füllt die Zwischenräume zwischen den Metallpartikeln 23 auf. Das Ergebnis zeigt Fig. 23. Das Füllmetall 60 wird allgemein vorzugsweise so ausgewählt, dass dessen Schmelzpunkt unterhalb der Transormationstemperatur $T_g$ des Glases und unterhalb der Schmelztemperatur der Metallpartikel 23 liegt. Weiterhin sollte auch eine hinreichend hohe Röntgenabsorbtion vorhanden sein, um den Röntgenkontrast mit dem Abschirmgitter zu verbessern. Gemäß noch einer Weiterbildung ist daher das Füllmetall so ausgewählt, dass es eine Röntgenabsorbtion aufweist, die mindestens halb so groß ist, wie die Röntgenabsorbtion von Wolfram.

Für das Füllmetall eignen sich damit insbesondere niedrigschmelzende Metalle oder Legierungen, etwa solche, die Zinn und/oder Blei enthalten. Der Schmelzpunkt des Füllmetalls 60 liegt vorzugsweise bei unter 300°C. Solche Eigenschaften werden unter anderem durch Lote erfüllt.

Damit das Füllmetall 60 in die Zwischenräume fließen kann, ist eine gute Benetzung der Metallpartikel 23 von Vorteil. Gemäß einer Weiterbildung ist dazu vorgesehen, dass die Metallpartikel 23, wie in Fig. 21 gezeigt, mit einer Beschichtung 230 versehen werden. Für eine bessere Benetzung kann dazu die Beschichtung 230 eine höhere Oberflächenenergie als Oberfläche der Metallpartikel 23 aufweisen. Wird Wolfram oder eine wolframhaltige Legierung für die Metallpartikel 23 verwendet, so weisen die Metallpartikel 23 bereits eine sehr hohe Oberflächenenergie auf, so dass eine gute Benetzung auch ohne eine Beschichtung 230 möglich ist. Bei der Darstellung der Fig. 23 ist gezeigt, dass ein Teil des Füllmetalls 60 auf der Oberfläche der Seite 5 verbleibt. Dieses Füllmetall 60 auf der Oberfläche kann beispielweise durch Polieren entfernt werden. Ist der Metallfilm nur sehr dünn, kann dieser gegebenenfalls auch verbleiben. Weiterhin kann, um das Entfernen des Füllmetalls 60 auf der Oberfläche zu erleichtern, oder gleich eine Benetzung zu vermeiden, die Oberfläche auf der Seite 5 poliert werden.

Weiterhin können das Füllmetall 60 oder die Metallpartikel 23 auch mit einem Flussmittel versehen sein, um die Benetzung zu verbessern. Allgemein wird mit dem Verfahren gemäß der Fig. 20 bis 23 ein Röntgenstrahlungs-absorbierendes Material 13 erhalten, welches in ein Füllmetall 60 eingebettete Metallpartikel 23 umfasst. Allgemein können sich durch die anhand der Fig. 20 bis 23 erläuterten Ausführungsformen weiterhin eines oder mehrere der folgenden Merkmale des röntgenabsorbierenden Materials 13 ergeben:

- Das Füllmetall hat einen Schmelzpunkt, der unter dem Schmelzpunkt der Metallpartikel 23 und unterhalb der Transformationstemperatur $T_g$ des Glases der Scheibe 3 liegt.
- unabhängig davon, ob die Metallpartikel 23 in ein Füllmetall 60 eingebettet sind, können die Metallpartikel 23 des Röntgenstrahlungs-absorbierenden Materials 13 eine Beschichtung 230 aufweisen, welche eine höhere Oberflächenenergie als das Material der Metallpartikel 23 hat. Eine solche Beschichtung 230 kann auch dazu dienen, die Rieselfähigkeit der Metallpartikel 23 zu erhöhen. Diese Weiterbildung kann auch besonders in Verbindung mit der Ausführungsform der Fig. 16 bis Fig. 19 von Vorteil sein. Hierzu eignen sich insbesondere silanisierte Metallpartikel 23. Gemäß noch einer Weiterbildung ist daher vorgesehen, dass das Röntgenstrahlungs-absorbierende Material 13 silanisierte Metallpartikel 23 enthält. Eine solche Beschichtung kann beispielsweise mit einem CVD-Prozess in einem Plasma abgeschieden werden. Auch andere Oberflächenmodifikationen können in einem Plasma erreicht werden. Daher ist gemäß einer Weiterbildung des Verfahrens vorgesehen, dass in die Gräben 11 ein Röntgenstrahlungs-absorbierendes Material 13 eingefüllt wird, welches Metallpartikel 23 enthält, wobei die Oberfläche der Metallpartikel 23 in einem Plasma modifiziert wird. Das Modifizieren kann vor dem Einfüllen, gegebenenfalls auch erst nach dem Befüllen durchgeführt werden.

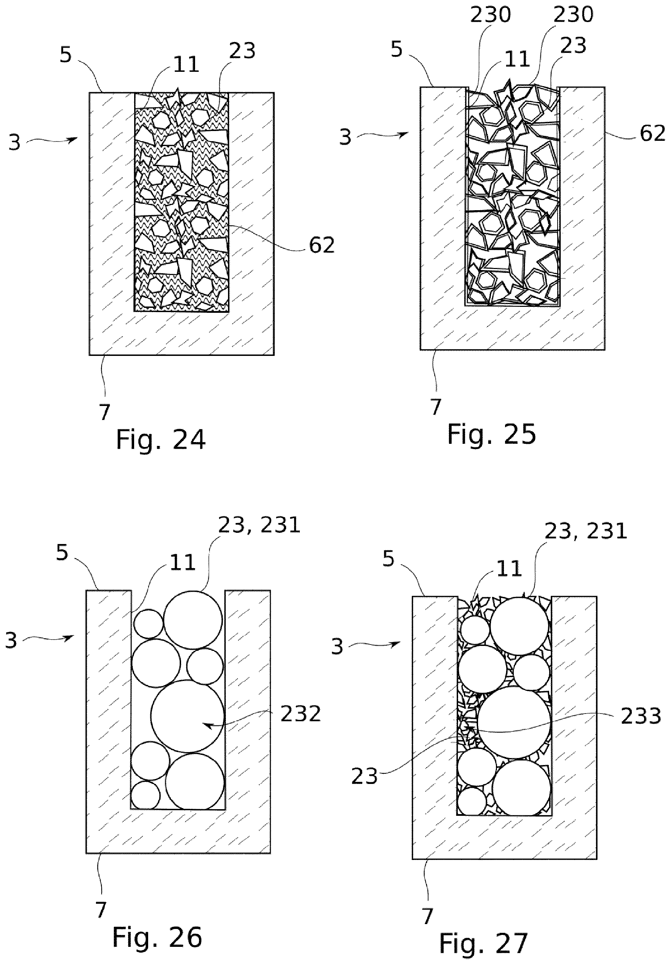

Anhand der Fig..24 und Fig. 25 werden Verfahrensschritte gemäß einer weiteren Ausführungsform erläutert, mit welcher die Zwischenräume in der Befüllung aufgefüllt oder geschlossen werden können.

Zunächst werden die Gräben 11 mit Metallpartikeln 23 aufgefüllt, so wie in Fig. 20 dargestellt. Anschließend wird eine Metalltinte 62 eingefüllt, welche, wie in Fig. 24 gezeigt, die Zwischenräume zwischen den Metallpartikeln 23 auffüllt. Wenn die Metalltinte 62 trocknet, hinterlässt diese auf den Metallpartikeln 23 und der Wandung der Gräben 11 wie in Fig. 25 dargestellt eine Beschichtung 230, die metallisch oder metallhaltig ist. Dieser Prozess kann gegebenenfalls ein- oder mehrmals wiederholt werden, um eine möglichst dichte Füllung zu erreichen. Als Metalle der Beschichtung 230 eignen sich wiederum solche, die eine hohe Röntgenabsorbtion aufweisen. Generell, unabhängig von dem Verfahren der Schichtabscheidung ist dazu gemäß einer Ausführungsform vorgesehen, dass die Gräben 11 mit Röntgenstrahlungs-absorbierendem Material 13 befüllt sind, welches Metallpartikel 23 umfasst, die eine Beschichtung 230 aufweisen, welche Röntgenstrahlung absorbiert. Dazu gelten insbesondere die oben genannte Kriterien für ein Röntgenstrahlung absorbierendes Material gemäß dieser Offenbarung, also ein Material, dessen Röntgenabsorptionskoeffizient mindestens um einen Faktor 3 größer ist, als der Röntgenabsorbtionskoeffizient des Materials der Scheibe für Röntgenstrahlung mit einer Energie von 69,5 keV und/oder ein Material mit einer Dichte, die mindestens viermal größer ist als die Dichte des Materials der Scheibe und/oder ein Material, welches zu mindestens 10 Gewichtsprozent Elemente mit einer Kernladungszahl von mindestens Z=56 aufweist.

Eine weitere Möglichkeit zur Abscheidung einer Röntgenstrahlung absorbierenden Beschichtung 230 auf den Metallpartikeln 23 und den Wandungen der Gräben 11 ist die Atomlagenabscheidung, auch als ALD (Atomic Layer Deposition)-Verfahren bezeichnet. Dabei werden alternierend verschiedene gasförmige Präkursoren eingeleitet, welche zur Reaktion mit den Oberflächen gebracht werden, wodurch sich jeweils sehr dünne, sogar monoatomare Schichten abscheiden. Sehr vorteilhaft können mit diesem Verfahren sogar Wolfram-Beschichtungen abgeschieden werden. Dazu ist es bekannt, als Precursoren $B_2H_6$ oder $SiH_4$ in Kombination mit $WF_6$ zu verwenden. Noch weitere Verfahren, um Röntgenstrahlung absorbierende Beschichtungen 230 abzuscheiden und damit die Zwischenräume zwischen den Metallpartikeln 23 wenigstens teilweise aufzufüllen, sind Tauchbeschichtung, etwa zur Herstellung von ITO- oder AZO-Schichten, sowie galvanisches Aufwachsen. Generell ist in Weiterbildung den verschiedenen Ausführungsformen des anhand der Fig. 24 und Fig. 25 erläuterten Verfahrens also vorgesehen, dass die Gräben 11 mit Metallpartikeln 23 gefüllt werden und nach dem Befüllen die Metallpartikeln 23 mit einer Röntgenstrahlungs-absorbierenden Beschichtung 230 versehen werden. Diese Verfahrensvarianten lassen sich auch mit anderen hier beschriebenen Verfahren zum Befüllen der Gräben 11, wie etwa dem Einfüllen einer Paste, die Glaspulver enthält, kombinieren. So könnten nach dem Trocknen der Paste auch hier die Metallpartikel beschichtet werden, oder es werden schichtweise verschiedene Verfahren kombiniert, so dass die Gräben 11 in verschiedenen Tiefen unterschiedlich aufgebaute Röntgenstrahlung absorbierende Materialien aufweisen. Diese Kombinierbarkeit gilt in gleicher Weise auch für die im Weiteren beschriebenen Verfahrensvarianten.

Die Fig. 26 und Fig. 27 zeigen eine weitere Ausführungsform eines Verfahrens zum Befüllen der Gräben 11 mit Metallpartikeln. Bei dieser Ausführungsform erfolgt die Befüllung der Gräben nacheinander mit Metallpulvern 232, 233 unterschiedlicher Körnung und Partikelform. Zunächst erfolgt, wie in Fig. 26 gezeigt, eine Befüllung mit einem Metallpulver 232 mit kugelförmigen Metallpartikeln 231. Diese sind vorzugsweise vergleichsweise groß, so dass große Zwischenräume zwischen den Metallpartikeln verbleiben. Solche kugelförmigen Metallpartikel 231 werden beispielsweise für den 3D-Druck eingesetzt. Die Kugelform ist beim 3D-Druck hilfreich, um eine hohe Rieselfähigkeit und damit die Möglichkeit, durch Abziehen sehr dünne Lagen loser Partikel auf dem zu druckenden Objekt herzustellen. Diese hohe Rieselfähigkeit ermöglicht entsprechend auch eine einfache Befüllung der Gräben 11. Metallpulver für den 3D-Druck mit kugelförmigen, beziehungsweise mindestens näherungsweise kugelförmigen Metallpartikeln 231 werden üblicherweise durch Zerstäubung hergestellt. Dazu wird ein Gas- oder Flüssigkeitsstrahl, typischerweise ein Wasserstrahl genutzt, um einen geschmolzenen Metallstrom in winzige Tröpfchen aufzubrechen. Die mindestens näherungsweise Kugelform der Partikel 231, wie sie gemäß dieser Ausführungsform vorgesehen sind, ergibt sich insbesondere durch den vorgenannten Zerstäubungsprozess. In einem zweiten Schritt wird wie in Fig. 27 gezeigt, ein Metallpulver 233 hinzugefüllt, welches im Vergleich zu den kugelförmigen Metallpartikeln 231 deutlich kleinere Metallpartikel 23 aufweist. Diese kleineren Metallpartikel sind aufgrund der großen Zwischenräume zwischen den kugelförmigen Metallpartikeln 231 in der Lage, diese Zwischenräume gut aufzufüllen. Anders als dargestellt können auch die kleineren Metallpartikel des später hinzugefügten Metallpulvers 233 kugelförmig sein. Dies ist ebenfalls aufgrund der guten Rieselfähigkeit solcher Partikel günstig.

Ohne Beschränkung auf bestimmte Beispiele ist in Bezug auf die Ausführungsform der Fig. 26, Fig. 27 ein Abschirmgitter 1 mit zumindest einem der folgenden Merkmale vorgesehen:

- das Röntgenstrahlungs-absorbierende Material umfasst kugelförmige Metallpartikel 231,
- das Röntgenstrahlungs-absorbierende Material umfasst zwei Metallpulver, die sich hinsichtlich der Partikelgröße der Metallpartikel 23, 231 unterscheiden, wobei die mittleren Partikelgrößen sich um mindestens einen Faktor zwei unterscheiden, wobei die größeren Metallpartikel 23 kugelförmig sind. Das Röntgenstrahlungs-absorbierende Material 13 weist damit insgesamt eine mindestens bimodale Partikelgrößenverteilung auf, die zwei Maxima aufweist. Das entsprechende Herstellungsverfahren ist dann dadurch gekennzeichnet, dass das Einfüllen des Röntgenstrahlungs-absorbierenden Materials 13 in die Gräben 11 das Einfüllen eines ersten Metallpulvers 232 mit kugelförmigen Metallpartikeln 231 und anschließendes Einfüllen eines zweiten Metallpulvers 233 umfasst, wobei die Metallpartikel 23 des zweiten Metallpulvers 233 eine kleinere mittlere Korngröße aufweisen, als die kugelförmigen Metallpartikel 231 des ersten Metallpulvers 232. Auch diese Ausführungsform, wie auch die anderen hierin beschriebenen Ausführungsformen betreffend das Befüllen der Gräben 11 ist unabhängig davon, wie die Gräben 11 in der Scheibe 3 erzeugt werden.

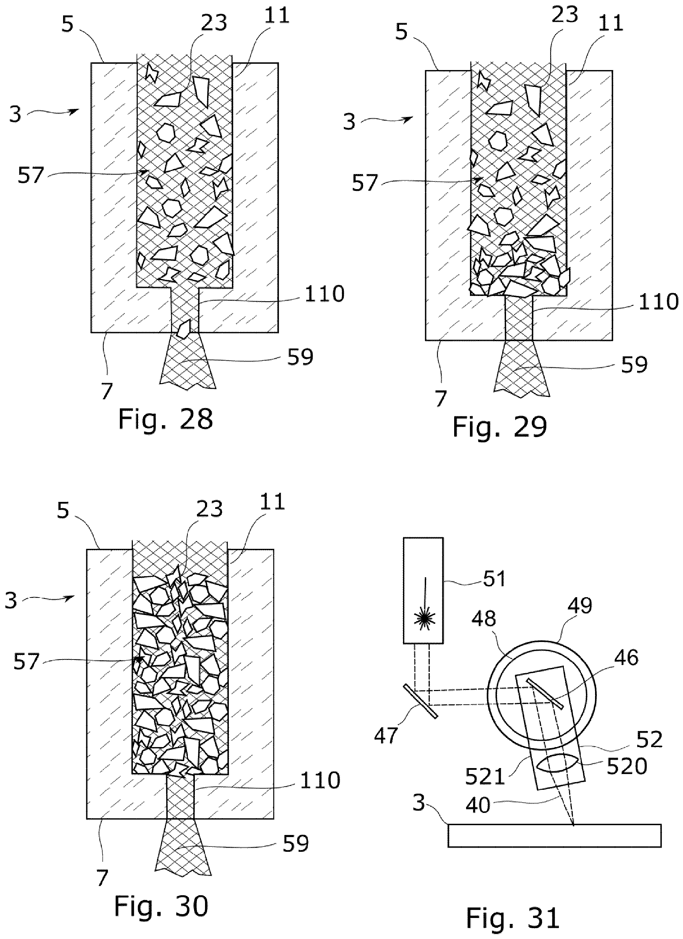

Die Fig. 28 bis Fig. 30 zeigen noch eine weitere Ausführungsform für die Befüllung der Gräben 11. Auch diese Ausführungsform kann mit anderen Befüllungsmethoden, wie etwa der zweistufigen Befüllung gemäß den Fig. 26, Fig. 27 kombiniert werden. Bei der Ausführungsform, wie sie anhand der Fig. 28 bis Fig. 30 erläutert wird, werden die Gräben mit einer Paste oder Dispersion 57 durchströmt, wobei sich die Metallpartikel 23 beim Durchströmen in den Gräben 11 absetzen, beziehungsweise dort abgelagert werden. Um diese Art der Befüllung durchführen zu können, ist in einer Weiterbildung der Scheibe 3 vorgesehen, dass deren Gräben 11 jeweils eine Bodenöffnung 110 aufweisen. Dies stellt eine Variante der bereits weiter oben beschriebenen Ausführungsform dar, gemäß welcher die Gräben 11 zumindest teilweise an beiden Seiten 5, 7 der Scheibe 3 offen sind.

Zur Befüllung werden die Gräben 11 mit einer Paste mit hohem Anteil an Dispersionsmedium 59, beziehungsweise einer Dispersion 57 durchströmt, so dass das Dispersionsmedium 59, wie in Fig. 28 gezeigt, an der Bodenöffnung 110 wieder austritt. Zumindest ein Teil der Metallpartikel 23 ist zu groß, um durch die Bodenöffnung 110 zu gelangen. Dadurch wird die Bodenöffnung 110 so blockiert, dass nur das Dispersionsmedium 59 die Öffnung passieren kann, während die Metallpartikel 23, wie beispielsweise Wolframpartikel ausgefiltert werden und im Graben 11 verbleiben. Eventuell können noch sehr kleine Partikel zunächst die Barriere an der Bodenöffnung 110 passieren. Diesen Zustand zeigt Fig. 29. Die Durchströmung wird dann solange fortgesetzt, bis sich die Kavität, beziehungsweise der Graben 11 hinreichend gefüllt hat. Den auf diese Weise gefüllten Graben 11 zeigt Fig. 30. Ohne Beschränkung auf das spezielle Beispiel der Fig. 28 bis Fig. 30 ist dazu gemäß einer Ausführungsform des Verfahrens vorgesehen, dass die Gräben 11 der Scheibe 3 mit Bodenöffnungen 110 versehen sind, wobei deren Querschnitt jeweils geringer als der Querschnitt des Grabens 11 ist, und wobei die Gräben 11 mit einer Metallpartikel 23 enthaltenden Dispersion 57 durchströmt werden, so dass das Dispersionsmedium 59 der Dispersion 57 an den Bodenöffnungen 110 austritt, wobei sich die Metallpartikel 23 an der Bodenöffnung 110 absetzen und diese für den Durchgang weiterer Metallpartikel 23 versperren, und wobei die Gräben durch weiteres Durchströmen mit der Dispersion 57 und Absetzen der Metallpartikel gefüllt werden.

Es ist dem Fachmann ersichtlich, dass die Erfindung nicht auf spezielle Ausführungsbeispiele, wie sie die Figuren zeigen, beschränkt ist, sondern in vielfältiger Weise im Rahmen dieser Offenbarung variiert werden kann. Insbesondere können dabei die verschiedenen Ausführungsformen auch miteinander kombiniert werden. So ist die Dispersion 57 in den Fig. 26 bis Fig. 30 nur mit Metallpartikeln 23 dargestellt. Die Dispersion 57 kann ebenso wie die in den zuvor beschriebenen Ausführungsformen der Paste auch andere Partikel, wie etwa Glaspartikel enthalten. Diese können nach dem Befüllen dann wie bereits beschrieben aufgeschmolzen werden, um das Röntgenstrahlungs-absorbierende Material 13 in den Gräben 11 zu verfestigen und mit den Gräben 11 fest zu verbinden. Das Herstellungsverfahren, insbesondere hinsichtlich der Befüllung der Gräben 11 kann weiterhin auch für andere Vorrichtungen als die hier beschriebenen Röntgenmasken verwendet werden. So kann eine Anordnung von strahlungsabsorbierenden Gräben auch für optische Einrichtungen, wie etwa flächig ausgedehnte Lichtleiter verwendet werden. In einem solchen Fall ist es dann auch nicht notwendig, dass die Füllung der Gräben 11 röntgenabsorbierend ist. Vielmehr kann eine an die spektrale Verteilung der Strahlung angepasste Strahlungsabsorption vorhanden sein. Gegebenenfalls können auch noch andere Eigenschaften, wie etwa eine hohe magnetische Permeabilität des Materials in den Gräben gegeben sein. So ist gemäß noch einer Ausführungsform vorgesehen, dass zumindest ein Teil der Metallpartikel ferromagnetisch sein kann. Dies könnte beispielsweise für eine selektive induktive Erwärmung verwendet werden. Allgemein ist daher in einer weiteren Ausführungsform ein Verfahren zur Herstellung eines Elements mit einer Anordnung von Gräben 11, insbesondere einem Gitter sich kreuzender Gräben vorgesehen, mit den wie oben bereits genannten Schritten:

- Bereitstellen einer Scheibe 3 mit einer ersten Seite 5 und einer der ersten Seite 5 gegenüberliegenden zweiten Seite 7, und Herstellen einer Anordnung aus zur ersten Seite 5 hin geöffneten Gräben 11, und Einfüllen eines strahlungsabsorbierenden Materials in die Gräben 11, wobei das Befüllen insbesondere mit einer Dispersion oder Paste mit Metallpartikeln 23 erfolgt. Die gesamte hier bezüglich des Abschirmgitters dargelegte Offenbarung, insbesondere auch bezüglich des Herstellungsverfahrens, der Form und Abmessung der Gräben 11, der Befüllung der Gräben, der Verfestigung, etwa durch Aufschmelzen eines Glaslots und des Materials der Füllung ist dabei auch auf solche anderen Elemente mit einer Anordnung von Gräben 11 anwendbar.

Nachfolgend wird in Ergänzung zur Erläuterung zu Fig. 6 ein Ausführungsbeispiel erläutert, mit welchem in die Scheibe 3 schräg verlaufende Gräben 11 und Vertiefungen 15 eingefügt werden. Fig. 31 zeigt dazu Teile einer Laser-Bearbeitungsvorrichtung zum Einfügen von filamentförmigen Schädigungen in die Scheibe als Weiterbildung der Ausführungsform der Fig. 6. Vorzugsweise und ohne Beschränkung auf das dargestellte Beispiel wird der Laserstrahl 40 über einen Drehspiegel 46 auf die Scheibe 3 gelenkt. Besonders bevorzugt ist ein zusätzlicher Umlenkspiegel 47 vorgesehen, so dass der Laser 51 wie in dem Beispiel der Fig. 6 senkrecht auf die Scheibe 3 abstrahlt. Es sind zwei durch Kreise symbolisierte Drehvorrichtungen, vorzugsweise Drehtische 48, 49 vorgesehen, mit denen der Drehspiegel 46 und die Fokussierungsoptik 52 rotierbar sind. Die Drehvorrichtungen sind koaxial zueinander angeordnet, so dass deren Drehachsen zusammenfallen. An einer der Drehvorrichtungen, beispielsweise Drehtisch 48, ist der Drehspiegel 46. Die andere Drehvorrichtung, beziehungsweise Drehtisch 49, dreht die Fokussierungsoptik, die vorzugsweise wie bei dem Beispiel der Fig. 6 eine Linse 520 umfasst. Um den Laserstrahl unter einem bestimmten Winkel $\Theta$ auf die Scheibe zu richten, wird die Fokussierungseinrichtung 52 entsprechend um diesen Winkel mit dem Drehtisch 49 rotiert, während der Drehspiegel 46 mit dem Drehtisch 48 um einen halb so großen Winkel in die gleiche Richtung gedreht wird. Die Kopplung der beiden Drehungen kann mechanisch oder auch elektronisch erfolgen. Zusätzlich kann wie bei dem Beispiel der Fig. 6 die Einstellung des Auftrefforts auf der Scheibe 3 mit einer in Fig. 31 der Einfachheit halber nicht dargestellten Positioniereinrichtung 53 vorgenommen werden.

Gemäß einem Ausführungsbeispiel ist die (Fokussier-)Linse 520 bikonvex geformt und weist eine hohe sphärische Aberration auf oder die Abbildungsoptik insgesamt weist eine hohe sphärische Aberration auf. Hierdurch wird ein elongierter Fokus in Form einer Fokallinie ausgebildet. Die Linse 520 kann auch entsprechend asphärisch oder sogar als Axikonlinse ausgebildet sein. In einer Variante wird zusätzlich das transversale Intensitätsprofil des Laserstrahls 40 geformt, so dass dieser ein transversales "Top-shape" Profil aufweist, d.h. über eine Strecke von 70%, bevorzugt 80%, besonders bevorzugt 85% des $1/e^2$-Strahldurchmessers des Laserstrahls beträgt die Abweichung der Intensität weniger als 30%, bev. weniger als 25%, bevorzugt weniger als 20%, besonders bevorzugt weniger als 10% vom Mittelwert der Intensität in diesem Bereich. In einem weiteren Ausführungsbeispiel ist auch die Intensität entlang der von der fokussierenden Linse 520 ausgebildeten, elongierten Fokallinie entlang der optischen Achse "Top-Shape"-förmig verteilt mit den zuvor angeführten Parametern. Der Drehtisch 49 kann in einem Winkelbereich von -40° bis +90° um die senkrechte Einfallsrichtung herum geschwenkt werden. Die Brennweite der Fokussierungsoptik 52, insbesondere der Linse 520 liegt im Bereich von 8 mm bis 30 mm, vorzugsweise im Bereich von 10 mm bis 24 mm, wobei der Fokus, beziehungsweise dessen Mitte in Strahlrichtung innerhalb der Scheibe 3 liegt. Der Strahldurchmesser vor Fokussierung liegt im Bereich von 8 mm bis 18 mm, vorzugsweise bei 12 mm. Gemäß einem Ausführungsbeispiel wird die Bearbeitung der Scheibe 3 aus Glas mit folgenden Parametern durchgeführt: Die Wellenlänge liegt im Bereich von 1000 nm bis 1100 nm, optional kann, insbesondere mittels Frequenzverdopplung eine Wellenlänge im Bereich von 500 nm bis 600 nm gewählt werden. Die Pulsdauer liegt im Bereich von 0,3 ps bis 10 ps. Die Repetitionsrate der Laserpulse beträgt 30 kHz bis 100 kHz. Der Laser wird im Burst-Modus mit 1 bis 8, vorzugsweise 2 bis 4 Pulsen je Burst betrieben. Die Pulsenergie liegt im Bereich von 5 $\mu$J bis 500 mJ, bevorzugt im Bereich 50 $\mu$J bis 50 mJ, besonders bevorzugt im Bereich 100 $\mu$J bis 10 mJ. Die Scheibe 3 wird - abhängig von der zu erzeugenden Geometrie - mit einer Geschwindigkeit von 100 mm/s - 500 mm/s abgerastert. Ein günstiger Abstand der filamentförmigen Schädigungen oder Modifikationen liegt im Bereich von 2 $\mu$m bis 20 $\mu$m, bevorzugt im Bereich 3 $\mu$m bis 10 $\mu$m.

Nach dem Einfügen der filamentförmigen Schädigungen oder Modifikationen wird die Scheibe 3 einem Ätzprozess ausgesetzt, um die Gräben 11 und Vertiefungen 15 herauszuarbeiten. Gegebenenfalls kann vor dem Ätzen noch ein Tempern erfolgen, um Spannungen im Material abzubauen. Dieses Annealing erfolgt im Bereich der Glasübergangstemperatur $T_g$, insbesondere leicht darüber, zum Beispiel bei $T_g$+20 °C. In einem Beispiel beträgt die Temperatur beim Tempern 545 °C bei einer Glasübergangstemperatur von 525 °C. Die Scheibe 3 wird dann einem sauren oder bevorzugt einem alkalischen Ätzmedium, im Speziellen einer KOH-Lösung, ausgesetzt. Die KOH-Konzentration liegt dabei im Bereich von 4 mol/l bis 22 mol/l, vorzugsweise im Bereich von 12 mol/l bis 18 mol/l. Das Ätzen erfolgt bei einer Temperatur von 60 °C bis 100 °C. Damit wird eine Ätzgeschwindigkeit von kleiner 0,5 $\mu$m pro Stunde bis 8 $\mu$m pro Stunde erreicht. Um die Gräben 11 und Vertiefungen 15 herauszuarbeiten, beträgt die Dauer des Ätzprozesses im Allgemeinen 2 bis 12 Stunden, typischerweise 4 bis 8 Stunden. Der Ätzprozess kann durch Ultraschall-Agitation unterstützt werden.

Bezugszeichenliste

| | |
|---|---|
| 1 | Abschirmgitter |
| 2 | bildgebendes Röntgengerät |
| 3 | Scheibe |
| 5 | erste Seite von 3 |
| 7 | zweite Seite von 3 |
| 8 | Dünnglas-Scheibe |
| 9 | Gitter |
| 10 | organische Verkapselung |
| 11 | Graben |
| 12 | Kanal |
| 13 | Röntgenstrahlungs- absorbierendes Material .... |
| 15 | Vertiefung |
| 16 | Bodenwandung von 15 |
| 17 | Mittenachse von 15 |
| 18 | Punktquelle |
| 19 | Wandung zwischen 11, 15 |
| 21 | Glaslot |
| 23 | Metallpartikel |
| 25 | Wand von 11 |
| 27 | Randbereich von 1 |

(fortgesetzt)

| 30 | Röntgenröhre |
|---|---|
| 31 | Anode |
| 32 | Kathode |
| 33 | Vakuumkolben |
| 34 | Objekt |
| 35 | Röntgenstrahl |
| 36 | gestreuter Röntgenstrahl |
| 39 | Detektor |
| 40 | Laserstrahl |
| 41 | filamentförmige Schädigung |
| 43 | erste Gruppe von filamentförmigen Schädigungen 41 |
| 44 | zweite Gruppe von filamentförmigen Schädigungen 41 |
| 45 | Ätzmedium |
| 46 | Drehspiegel |
| 47 | Umlenkspiegel |
| 48, 49 | Drehtisch |
| 50 | Laser-Bearbeitungsvorrichtung |
| 51 | Ultrakurzpulslaser |
| 52 | Fokussierungsoptik |
| 53 | Positioniereinrichtung |
| 54 | Auftreffpunkt von 40 |
| 55 | Recheneinrichtung |
| 57 | Dispersion |
| 59 | Dispersionsmedium |
| 60 | Füllmetall |
| 62 | Metalltinte |
| 110 | Bodenöffnung in 11 |
| 230 | Beschichtung |
| 231 | kugelförmiger Metallpartikel |
| 232, 233 | Metallpulver |
| 520 | Linse |
| 521 | Tubus |

**Patentansprüche**

1. Abschirmgitter (1) gegen ionisierende Streustrahlung, insbesondere für eine bildgebende Röntgenvorrichtung, umfassend

- eine Scheibe (3) mit einer ersten Seite (5) und einer der ersten Seite (5) gegenüberliegenden zweiten Seite (7), wobei die Scheibe (3)
- eine Anordnung von Vertiefungen (15) aufweist, welche zur zweiten Seite (7) der Scheibe (3) hin geöffnet sind, und wobei

- die Scheibe (3) ein Gitter (9) aus zur ersten Seite (5) hin geöffneten Gräben (11) aufweist, wobei
- die Gräben (11) mit einem Röntgenstrahlungs- absorbierenden Material (13) gefüllt sind, und wobei
- die Gräben (11) von einer der Seiten (5, 7) aus betrachtet zwischen den Vertiefungen (15) und in einem Abstand zu den Vertiefungen (15) verlaufen, so dass zwischen den Vertiefungen und den Gräben (11) Wandungen (19) verbleiben.

2. Abschirmgitter (1) gemäß dem vorstehenden Anspruch, **dadurch gekennzeichnet, dass** die Scheibe als Glasscheibe ausgebildet ist.

3. Abschirmgitter (1) gemäß dem vorstehenden Anspruch, **gekennzeichnet durch** zumindest eines der folgenden Merkmale:

   - die Gräben (11) weisen ein Verhältnis von Tiefe zu Breite von mindestens 40:1 auf,
   - die Tiefe der Gräben beträgt mindestens 1,5 Millimeter,
   - der Winkel der Wand (25) eines Grabens (11) oder einer Vertiefung weicht um weniger als 5° vom Sollwinkel, insbesondere von der Richtung der Mittenachse (17) ab,
   - der Mitte-zu-Mitte-Abstand zweier benachbarter Gräben ist kleiner als deren Tiefe, vorzugsweise kleiner um mindestens einen Faktor drei.
   - die Breite der Gräben (11) unterscheidet sich von der Breite der Vertiefungen (15) um maximal einen Faktor 2
   - die Breite der Gräben (11) beträgt höchstens 100 $\mu$m, vorzugsweise höchstens 50 $\mu$m.

4. Abschirmgitter (1) gemäß einem der vorstehenden Ansprüche, **dadurch gekennzeichnet, dass** die Mittenachsen (17) der Vertiefungen (15) und/oder der Gräben (11) auf eine gemeinsame virtuelle Punktquelle (18) gerichtet sind

5. Abschirmgitter (1) gemäß einem der vorstehenden Ansprüche, **gekennzeichnet durch** zumindest eines der folgenden Merkmale:

   - das Röntgenstrahlungs- absorbierende Material (13) umfasst ein an- oder aufgeschmolzenes Glas,
   - das Röntgenstrahlungs-absorbierende Material (13) umfasst ein blei- und/oder bismuthhaltiges Glas
   - das Röntgenstrahlungs-absorbierende Material (13) enthält in einem an- oder aufgeschmolzenen Material, vorzugsweise einem Glas eingebettete Partikel, insbesondere Metallpartikel (23) und/oder mineralische Partikel und/oder keramische Partikel,
   - das Röntgenstrahlungs-absorbierende Material (13) weist eine Dichte von mindestens 9 g/cm$^3$, vorzugsweise mindestens 11 g/cm$^3$ auf,
   - die Dichte des Röntgenstrahlungs-absorbierenden Materials (13) ist mindestens viermal größer ist als die Dichte des Materials der Scheibe (3),
   - das Röntgenstrahlungs-absorbierende Material (13) weist zu mindestens 10 Gewichtsprozent Elemente mit einer Kernladungszahl von mindestens Z = 56, vorzugsweise zu mindestens 25 Gewichtsprozent auf,
   - das Röntgenstrahlungs-absorbierende Material (13) umfasst Partikel eines Wolfram enthaltenden Materials, vorzugsweise in Form von metallischem Wolfram, einer Wolfram-Legierung und/ oder eines Wolfram-haltigen Minerals und/oder einer Wolfram-haltigen Keramik,
   - die Differenz der linearen Temperaturausdehnungskoeffizienten von röntgenabsorbierendem Material (13) und dem Material der Scheibe (3) ist im Betrag kleiner als 3 ppm/K,
   - der Füllgrad des Röntgenstrahlungs-absorbierenden Materials (13) in den Gräben (11) beträgt mindestens 40 Vol%, vorzugsweise mindestens 60 Vol%,
   - das Glas des Röntgenstrahlungs-absorbierenden Materials (13) hat eine Glasübergangstemperatur $T_g$, die mindestens 160 °C, vorzugsweise mindestens 220 °C niedriger ist als die Glasübergangstemperatur $T_g$ des Glases der Scheibe 3,
   - das Glas des Röntgenstrahlungs-absorbierenden Materials (13) hat eine Verarbeitungstemperatur, bei der dessen Viskosität bei 10$^4$ dPa·s ist, die um mindestens 100 °C niedriger liegt, als die Verarbeitungstemperatur des Glases der Scheibe (3),
   - das Glas des Röntgenstrahlungs-absorbierenden Materials (13) hat eine Verarbeitungstemperatur, die niedriger ist als die Temperatur des Erweichungspunkts des Glases der Scheibe (3), wobei der Erweichungspunkt durch eine Viskosität von 10$^{7,6}$ dPa·s definiert ist.
   - das Röntgenstrahlungs-absorbierende Material (13) enthält zumindest eine Komponente in Partikelform, welche in zumindest einem Teil des Temperaturbereichs zwischen 0 °C und 200 °C einen linearen Temperaturausdehnungskoeffizienten aufweist, der kleiner als 1 ppm/K oder negativ ist.

6. Abschirmgitter (1) gemäß einem der vorstehenden Ansprüche, **gekennzeichnet durch** zumindest eines der folgenden Merkmale:

   - die Gräben (11) sind zumindest teilweise an beiden Seiten (5, 7) der Scheibe (3) offen,
   - die Gräben (11) weisen eine Bodenöffnung (110) auf.

7. Abschirmgitter (1) gemäß einem der vorstehenden Ansprüche, **gekennzeichnet durch** zumindest eines der folgenden Merkmale:

   - das Röntgenstrahlungs-absorbierende Material (13) umfasst Partikel in Form einer Mischung mindestens zweier Pulver, insbesondere Metallpulver, und/ oder Mineralpulver und/oder Keramikpulver mit unterschiedlichen d50-Werten,
   - das Röntgenstrahlungs-absorbierende Material (13) enthält Partikel, die eine Partikelgrößenverteilung aufweisen, für welche der Exponent n einer an die Partikelgrößenverteilung der Metallpartikel (23) angepassten

$$F(d) = 100 * \left(\frac{d}{d_{max}}\right)^n$$

   Andreasen-Verteilung der Partikeldurchmesser d, kleiner als n = 0,33, vorzugsweise kleiner als n = 0,28 ist, wobei $d_{max}$ der maximale Partikeldurchmesser der angepassten Andreasen-Verteilung ist,
   - die Partikelgrößenverteilung der Partikel im Röntgenstrahlungs-absorbierenden Material (13) ist bimodal oder multimodal,
   - das Röntgenstrahlungs-absorbierende Material (13) umfasst in ein Füllmetall (60) eingebette Metallpartikel (23), wobei das Füllmetall (60) einen Schmelzpunkt hat, der unter dem Schmelzpunkt der Metallpartikel (23) und unterhalb der Transformationstemperatur $T_g$ des Glases der Scheibe (3) liegt;
   - das Röntgenstrahlungs-absorbierende Material (13) umfasst Metallpartikel (23), welche eine Beschichtung (230) aufweisen, welche vorzugsweise eine höhere Oberflächenenergie als das Material der Metallpartikel (23) hat, oder Röntgenstrahlung absorbiert,
   - das Röntgenstrahlungs-absorbierende Material (13) enthält silanisierte Metallpartikel 23,
   das Röntgenstrahlungs-absorbierende Material umfasst kugelförmige Metallpartikel 231,
   - das Röntgenstrahlungs-absorbierende Material umfasst zwei Metallpulver, die sich hinsichtlich der Partikelgröße der Metallpartikel (23, 231) unterscheiden, wobei die mittleren Partikelgrößen sich um mindestens einen Faktor zwei unterscheiden, wobei die größeren Metallpartikel (23) kugelförmig sind..

8. Abschirmgitter (1) gemäß einem der vorstehenden Ansprüche, **gekennzeichnet durch** zumindest eines der folgenden Merkmale:

   - auf wenigstens einer Seite (5, 7) der Scheibe (3) ist eine Dünnglas-Scheibe (8) befestigt,
   - auf zumindest einem Teil der Oberfläche der Scheibe (3) ist eine organische Verkapselung (10) aufgebracht.

9. Bildgebende Röntgenvorrichtung, insbesondere Computertomograph (2) mit einer Röntgenquelle und einem vor einem Röntgendetektor (39) zur Detektion der von der Röntgenquelle emittierten Röntgenstrahlung angeordneten Abschirmgitter (1) gemäß einem der vorstehenden Ansprüche.

10. Verfahren zur Herstellung eines Abschirmgitters (1) gegen Röntgen-Streustrahlung gemäß einem der vorstehenden Ansprüche, mit den Schritten:

   - Bereitstellen einer Scheibe (3) mit einer ersten Seite (5) und einer der ersten Seite (5) gegenüberliegenden zweiten Seite (7), und
   - Bestrahlen der Scheibe (3) mit einem Laserstrahl (40), wobei das Material der Scheibe (3) für den Laserstrahl (40) transparent ist, so dass der Laserstrahl (40) in die Scheibe (3) eindringt, und wobei
   - der Laserstrahl (40) entlang seines Pfades durch die Scheibe (3) filamentförmige Schädigungen (41) hinterlässt, und wobei die filamentförmigen Schädigungen (41) so eingefügt werden, dass eine erste Gruppe (43) der filamentförmigen Schädigungen (41) an der ersten Seite (5) und eine zweite Gruppe (44) der filamentförmigen Schädigungen (41) an der zweiten Seite (7) endet,
   - Entfernen des Materials der Scheibe (3) im Bereich der ersten und zweiten Gruppen (43, 44) von filamentförmigen Schädigungen (41) durch Ätzen der Scheibe (3) mit einem Ätzmedium (45), so dass
   - durch das Entfernen des Materials im Bereich der zweiten Gruppe (44) eine Anordnung von Vertiefungen (15),

welche zur zweiten Seite (7) der Scheibe (3) hin geöffnet sind, und
- durch das Entfernen des Materials im Bereich der ersten Gruppe (43) ein Gitter (9) aus zur ersten Seite (5) hin geöffneten Gräben (11) erzeugt wird, und wobei dann
- in die Gräben (11) ein Röntgenstrahlungs-absorbierendes Material (13) gefüllt wird.

11. Verfahren gemäß dem vorstehenden Anspruch, **dadurch gekennzeichnet, dass** ein pulverförmiges Glas, vorzugsweise ein Glaslot (21) als Bestandteil des Röntgenstrahlungs-absorbierenden Materials (13), vorzugsweise eine Mischung eines pulverförmigen Glases mit Partikeln in die Gräben (11) eingefüllt wird, und wobei das Glaslot (21) auf- oder angeschmolzen wird, so dass ein festes Röntgenstrahlungs- absorbierendes Material (13) erzeugt wird, welches an den Wänden der Gräben (11) anhaftet.

12. Verfahren gemäß einem der zwei vorstehenden Ansprüche, **dadurch gekennzeichnet, dass** die filamentförmigen Schädigungen (41) zumindest teilweise schräg zu einer der Seiten (5, 7) der Scheibe (3) eingefügt werden.

13. Verfahren gemäß einem der drei vorstehenden Ansprüche, **dadurch gekennzeichnet, dass** zumindest ein Teil, vorzugsweise die filamentförmigen Schädigungen beider Gruppen (43, 44) innerhalb der Scheibe (3) enden.

14. Verfahren gemäß einem der vier vorstehenden Ansprüche, **dadurch gekennzeichnet, dass** zumindest ein Teil der filamentförmigen Schädigungen (41) beider Gruppen (43, 44) unter Einstrahlung des Laserstrahls (40) auf die gleiche Seite (5, 7) der Scheibe (3) und Änderung der Lage des Fokus des Laserstrahls (40) relativ zur Scheibe und in der Richtung entlang des Laserstrahls (40) erzeugt wird.

15. Verfahren gemäß einem der fünf vorstehenden Ansprüche, bei welchem eine Paste bereitgestellt wird, welche Glaspartikel und Metallpartikel (23) enthält, wobei die Paste in die Gräben (11) gefüllt wird, und wobei die Scheibe (3) aufgeheizt wird, so dass die Glaspartikel erweichen und ein Röntgenstrahlungs-absorbierendes Material (13) mit in Glas eingebetteten Partikeln erhalten wird.

16. Verfahren gemäß einem der sechs vorstehenden Ansprüche, umfassend die Schritte:

   - Messen oder Berechnen einer Packungsdichte einer Mischung eines gemahlenen Glases mit zumindest einem weiteren Pulver,
   - Berechnen oder Messen der Packungsdichte der Mischung,
   - zumindest einmaliges Berechnen oder Messen der Packungsdichte einer Mischung mit variiertem Mischungsverhältnis,
   - aus den zumindest zwei Ergebnissen der Berechnung oder Messung Auswählen oder Bestimmen eines Mischungsverhältnisses für eine Zubereitung zum Befüllen der Gräben (11) und
   - Herstellung einer Zubereitung mit dem Mischungsverhältnis.

17. Verfahren gemäß einem der Ansprüche 10 bis 16, **dadurch gekennzeichnet, dass** die Gräben (11) mit Metallpartikeln (23) gefüllt werden und nach dem Befüllen die Metallpartikel (23) mit einer Röntgenstrahlungs-absorbierenden Beschichtung (230) versehen werden.

18. Verfahren gemäß einem der Ansprüche 10 bis 17, **dadurch gekennzeichnet, dass** das Einfüllen des Röntgenstrahlungs-absorbierenden Materials (13) in die Gräben (11) das Einfüllen eines ersten Metallpulvers (232) mit kugelförmigen Metallpartikeln (231) und anschließendes Einfüllen eines zweiten Metallpulvers (233) umfasst, wobei die Metallpartikel (23) des zweiten Metallpulvers (233) eine kleinere mittlere Korngröße aufweisen, als die kugelförmigen Metallpartikel (231) des ersten Metallpulvers (232).

19. Verfahren gemäß einem der Ansprüche 10 bis 18, wobei die Gräben (11) der Scheibe (3) mit Bodenöffnungen (110) versehen sind, wobei deren Querschnitt jeweils geringer als der Querschnitt des Grabens (11) ist, und wobei die Gräben (11) mit einer Metallpartikel (23) enthaltenden Dispersion (57) durchströmt werden, so dass das Dispersionsmedium (59) der Dispersion (57) an den Bodenöffnungen (110) austritt, wobei sich die Metallpartikel (23) an der Bodenöffnung (110) absetzen und diese für den Durchgang weiterer Metallpartikel (23) versperren, und wobei die Gräben (11) durch weiteres Durchströmen mit der Dispersion (57) und Absetzen der Metallpartikel (23) gefüllt werden.

Fig. 1

Fig. 2

Fig. 3

Fig. 4

Fig. 5

Fig. 6

EP 4 231 316 A1

Fig. 7

Fig. 8

Fig. 9

Fig. 10

Fig. 11

Fig. 12

Fig. 13

EP 4 231 316 A1

Fig. 14

Fig. 15

Fig. 16

Fig. 17

Fig. 18

Fig. 19

Fig. 20

Fig. 21

Fig. 22

Fig. 23

Fig. 24

Fig. 25

Fig. 26

Fig. 27

Fig. 28

Fig. 29

Fig. 30

Fig. 31

Europäisches Patentamt

European Patent Office

Office européen des brevets

**EUROPÄISCHER RECHERCHENBERICHT**

Nummer der Anmeldung

EP 23 15 6813

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | JP 2013 181917 A (FUJIFILM CORP) 12. September 2013 (2013-09-12) * Zusammenfassung; Abbildungen * | 1,3-5,9 | INV. G21K1/02 B23K26/0622 B23K26/53 C03C23/00 |
| X | JP S53 12679 A (HITACHI CABLE; HITACHI MEDICAL CORP) 4. Februar 1978 (1978-02-04) * Zusammenfassung * | 1,3,5,6,9 | |
| X | US 2011/317819 A1 (SHAW JEFFREY JON [US] ET AL) 29. Dezember 2011 (2011-12-29) * Abb. 1 bis 5 und zugehöriger Text * | 1,3-5,9 | |
| X | US 2 801 671 A (VAUGHN EVERETT W ET AL) 6. August 1957 (1957-08-06) * Abb. 1, 3 und zugehöriger Text * | 1,3-6,9 | |
| X | SU 441 019 A1 (?) 30. August 1974 (1974-08-30) * Zusammenfassung; Abbildungen * | 1-6,9 | |
| X | LEHMANN V ET AL: "MEMS techniques applied to the fabrication of anti-scatter grids for X-ray imaging", SENSORS AND ACTUATORS A: PHYSICAL, ELSEVIER BV, NL, Bd. 95, Nr. 2-3, 1. Januar 2002 (2002-01-01), Seiten 202-207, XP004326760, ISSN: 0924-4247, DOI: 10.1016/S0924-4247(01)00737-3 * Abb. 1 und zugehöriger Text * | 1,3,5,8,9 | RECHERCHIERTE SACHGEBIETE (IPC) G21K B23K C03C |

Der vorliegende Recherchenbericht wurde für alle Patentansprüche erstellt

| Recherchenort | Abschlußdatum der Recherche | Prüfer |
|---|---|---|
| München | 6. Juli 2023 | Krauss, Jan |

EPO FORM 1503 03.82 (P04C03)

# EP 4 231 316 A1

## ANHANG ZUM EUROPÄISCHEN RECHERCHENBERICHT
## ÜBER DIE EUROPÄISCHE PATENTANMELDUNG NR.

EP 23 15 6813

06-07-2023

| Im Recherchenbericht angeführtes Patentdokument | Datum der Veröffentlichung | Mitglied(er) der Patentfamilie | Datum der Veröffentlichung |
|---|---|---|---|
| JP 2013181917 A | 12-09-2013 | JP 2013181917 A | 12-09-2013 |
| | | WO 2013129309 A1 | 06-09-2013 |
| JP S5312679 A | 04-02-1978 | JP S5312679 A | 04-02-1978 |
| | | JP S5933237 B2 | 14-08-1984 |
| US 2011317819 A1 | 29-12-2011 | CN 102389320 A | 28-03-2012 |
| | | DE 102011050963 A1 | 12-01-2012 |
| | | JP 5977489 B2 | 24-08-2016 |
| | | JP 2012005839 A | 12-01-2012 |
| | | US 2011317819 A1 | 29-12-2011 |
| US 2801671 A | 06-08-1957 | KEINE | |
| SU 441019 A1 | 30-08-1974 | KEINE | |

EPO FORM P0461

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2007034352 A2
- US 2016163408 A1
- US 5581592 A

- DE 102017101673
- DE 102018110211
- EP 2021077030 W